# EUROPÄISCHE PATENTSCHRIFT

(11) **EP 0 994 865 B1**
(45) Veröffentlichungstag und Bekanntmachung des Hinweises auf die Patenterteilung: **06.03.2002**
(21) Anmeldenummer: 98938687.5
(22) Anmeldetag: 03.07.1998
(51) Int. Cl.: C07D 249/12, C07D 249/08, C07D 401/12, C07D 401/14, C07D 403/12, C07D 403/14, C07D 405/14, C07D 409/14, C07D 413/14, C07D 417/14, A61K 31/33, A61K 31/41

(54) **TRIAZOLVERBINDUNGEN UND DEREN VERWENDUNG ALS DOPAMIN-D 3-LIGANDEN**
TRIAZOLE COMPOUNDS AND THE USE THEREOF AS DOPAMINE-D 3-LIGANDS
COMPOSES DE TRIAZOLE ET LEUR UTILISATION COMME LIGANDS DE LA DOPAMINE D 3

(30) Priorität: 07.07.1997 DE 19728996
(43) Veröffentlichungstag der Anmeldung: 26.04.2000
(73) Patentinhaber: BASF AKTIENGESELLSCHAFT, 67056 Ludwigshafen (DE)
(72) Erfinder: STARCK, Dorothea, D-67059 Ludwigshafen (DE); BLANK, Stefan, D-67065 Ludwigshafen (DE); TREIBER, Hans-Jörg, D-68782 Brühl (DE); UNGER, Liliane, D-67065 Ludwigshafen (DE); NEUMANN-SCHULTZ, Barbara, D-68526 Ladenburg (DE); LE BRIS, Theophile-Marie, D-67273 Bobenheim am Berg (DE); TESCHENDORF, Hans-Jürgen, D-67373 Dudenhofen (DE); WICKE, Karsten, D-67112 Altrip (DE)
(74) Vertreter: Kinzebach, Werner, Dr.
(86) Internationale Anmeldenummer: EP9804138
(87) Internationale Veröffentlichungsnummer: WO9902503

(56) Entgegenhaltungen:
- WO-A-96/02520
- WO-A-97/25324
- US-A- 4 338 453
- US-A- 4 577 020
- BROWNE E J: "N-Unsubstituted 1,2,4-triazole-3-aldehydes" TETRAHEDRON LETTERS, Nr. 12, März 1970, Seiten 943-4, XP002082579
- DATABASE CROSSFIRE Beilstein Informationssysteme GmbH, Frankfurt DE XP002082581 & AUST. J. CHEM., Bd. 24, 1971, Seiten 393-403,
- DATABASE CROSSFIRE Beilstein Informationssysteme GmbH, Frankfurt DE XP002082582 & AUST. J. CHEM., Bd. 26, 1973, Seite 1809, 1811, 1813
- CZARNOCKA-JANOWICZ A ET AL: "Synthesis and pharmacological activity of 5-substituted-s-triazole-3-thiols" DIE PHARMAZIE, Bd. 46, Nr. 2, 1991, Seiten 109-112, XP002082580

## Beschreibung

Die Erfindung betrifft Triazolverbindungen und die Verwendung derartiger Verbindungen. Die erwähnten Verbindungen besitzen wertvolle therapeutische Eigenschaften und sind zur Behandlung von Erkrankungen brauchbar, die auf Dopamin-D₃-Rezeptorliganden ansprechen.

Verbindungen der hier in Rede stehenden Art mit physiologischer Aktivität sind bereits bekannt. Die US-A 4,338,453; 4,408,049 und 4,577,020 beschreiben Triazolverbindungen, welche anti-allergische oder anti-psychotische Aktivität besitzen. Die DE-A 44 25 144 und die WO 97/25324 beschreiben Triazolverbindungen, die auf Dopamin-D₃-Rezeptorliganden ansprechen. Verbindungen des gleichen strukturellen Typs, jedoch mit anderen Heterocyclen anstelle des Triazolrings sind in DE-A-44 25 146, DE-A-44 25 143 und DE-A-44 25 145 beschrieben.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin.

Es liegen gesicherte Erkenntnisse über die Anwesenheit von Dopamin und dessen physiologische Funktion als Neurotransmitter vor. Auf Dopamin ansprechende Zellen stehen im Zusammenhang mit der Etiologie von Schizophrenie und der Parkinson'schen Krankheit. Die Behandlung dieser und anderer Erkrankungen erfolgt mit Arzneimitteln, die mit den Dopaminrezeptoren in Wechselwirkung treten.

Bis 1990 waren zwei Subtypen von Dopaminrezeptoren pharmakologisch klar definiert, nämlich die D₁- und D₂-Rezeptoren.

In jüngerer Zeit wurde ein dritter Subtyp gefunden, nämlich der D₃-Rezeptor, der einige Effekte der Antipsychotika zu vermitteln scheint. (J.C. Schwartz et al., The Dopamine D₃ Receptor as a Target for Antipsychotics, in Novel Antipsychotic Drugs, H.Y. Meltzer, Ed. Raven Press, New York 1992, Seiten 135-144)

D₃-Rezeptoren werden hauptsächlich im limbischen System exprimiert. Es wird daher angenommen, daß ein selektiver D₃-Antagonist wohl die antipsychotischen Eigenschaften der D₂-Antagonisten, nicht aber ihre neurologischen Nebenwirkungen haben sollte. (P.

Solokoff et al., Localization and Function of the D₃ Dopamine Receptor, Arzneim. Forsch./Drug Res. 42(1), 224 (1992); P. Solokoff et al. Molecular Cloning and Characterization of a Novel Dopamine Receptor (D₃) as a Target for Neuroleptics, Nature, 347, 146 (1990)).

Überraschenderweise wurde nun gefunden, daß bestimmte Triazolverbindungen eine hohe Affinität zum Dopamin-D₃-Rezeptor und eine geringe Affinität zum D₂-Rezeptor aufweisen. Es handelt sich somit um selektive D₃-Liganden.

Gegenstand der vorliegenden Erfindung sind daher die Verbindungen der allgemeinen Formel I: 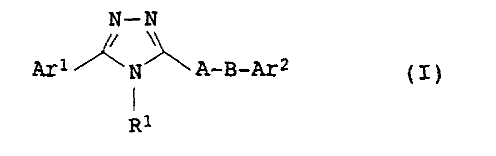 worin
- Ar¹: für Phenyl, Naphthyl oder einen 5- oder 6-gliedrigen heterocyclischen aromatischen Ring mit 1, 2, 3 oder 4 Heteroatomen, die unabhängig voneinander ausgewählt sind unter O, S und N, steht, wobei Ar¹ gegebenenfalls 1, 2, 3 oder 4 Substituenten aufweist, die unabhängig voneinander ausgewählt sind unter C₁-C₆-Alkyl, das gegebenenfalls durch OH, OC₁-C₆-Alkyl, Halogen oder Phenyl substituiert ist, C₁-C₆-Alkoxy, C₂-C₆-Alkenyl, C₂-C₆-Alkinyl, C₃-C₆-Cycloalkyl, Halogen, CN, COOR², NR²R², NO₂, SO₂R², SO₂NR²R² oder Phenyl, das gegebenenfalls durch C₁-C₆-Alkyl, OC₁-C₆-Alkyl, NR²R², CN, CF₃, CHF₂, oder Halogen substituiert ist, und wobei der erwähnte heterocyclische, aromatische Ring gegebenenfalls mit einem Phenylring kondensiert sein kann;
- A: für geradkettiges oder verzweigtes C₄-C₁₀-Alkylen oder geradkettiges oder verzweigtes C₃-C₁₀-Alkylen steht, das wenigstens eine Gruppe Z umfasst, die ausgewählt ist unter O, S, NR², CONR², COO, CO, einer Doppel- oder Dreifachbindung,
- B: für einen Rest der Formel steht: 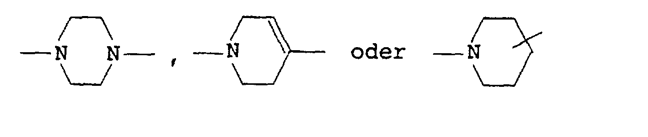 oder wenn Ar¹ für den 5- oder 6-gliedrigen, heterocyclischen aromatischen Ring, der wie angegeben substituiert sein kann, steht, B auch für einen Rest der Formeln 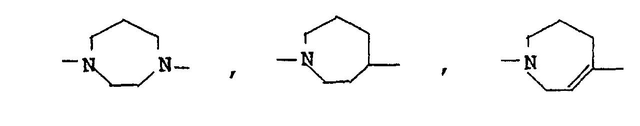 oder 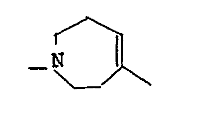 stehen kann,
- Ar²: für Phenyl, Pyridyl, Pyrimidinyl oder Triazinyl steht, wobei Ar² gegebenenfalls ein bis vier Substituenten aufweisen kann, die unabhängig voneinander ausgewählt sind unter OR², C₁-C₆-Alkyl, C₂-C₆-Alkenyl, C₂-C₆-Alkinyl, C₁-C₆-Alkoxy-C₁-C₆-alkyl, Halogen-C₁-C₆-alkyl, Halogen-C₁-C₆-alkoxy, Halogen, CN, NO₂, SO₂R², NR²R², SO₂NR²R², einem 5- oder 6-gliedrigen carbocyclischen, aromatischen oder nicht-aromatischen Ring und einem 5- oder 6-gliedrigen, heterocyclischen aromatischen oder nicht-aromatischen Ring mit 1 oder 2 Heteroatomen, die ausgewählt sind unter O, S und N, wobei der carbocyclische oder heterocyclische Ring gegebenenfalls durch C₁-C₆-Alkyl, Phenyl, Phenoxy, Halogen, OC₁-C₆-Alkyl, OH, NO₂ oder CF₃ substituiert und/oder mit einem Phenylring kondensiert sein kann und wobei Ar² gegebenenfalls mit einem carbocyclischen oder heterocyclischen Ring der oben definierten Art kondensiert sein kann,
- R¹: für H, C₃-C₆-Cycloalkyl oder C₁-C₆-Alkyl, das gegebenenfalls durch OH, OC₁-C₆-Alkyl oder Phenyl substituiert ist, steht;
- die Reste R²,: die gleich oder verschieden sein können, für H oder C₁-C₆-Alkyl, das gegebenenfalls durch OH, OC₁-C₆-Alkyl oder Phenyl substituiert ist, stehen;
sowie deren Salze mit physiologisch verträglichen Säuren.

Bei den erfindungsgemäßen Verbindungen handelt es sich um selektive Dopamin-D₃-Rezeptor-Liganden, die regioselektiv im limbischen System angreifen und aufgrund ihrer geringen Affinität zum D₂-Rezeptor nebenwirkungsärmer als die klassischen Neuroleptika sind, bei denen es sich um D₂-Rezeptorantagonisten handelt. Die Verbindungen sind daher zur Behandlung von Erkrankungen brauchbar, die auf Dopamin-D₃-Rezeptorantagonisten bzw. -agonisten ansprechen, z.B. zur Behandlung von Erkrankungen des zentralen Nervensystems insbesondere Schizophrenie, Depressionen, Neurosen, Psychosen, Parkinson und Angstzuständen.

Im Rahmen der vorliegenden Erfindung besitzen die nachfolgenden Ausdrücke die anschließend angegebenen Bedeutungen:

Alkyl (auch in Resten wie Alkoxy, Alkylamino etc.) bedeutet eine geradkettige oder verzweigte Alkylgruppe mit 1 bis 6 Kohlenstoffatomen und insbesondere 1 bis 4 Kohlenstoffatomen. Die Alkylgruppe kann einen oder mehrere Substituenten aufweisen, die unabhängig voneinander ausgewählt sind unter OH, OC₁-C₆-Alkyl, Halogen oder Phenyl.

Beispiele für eine Alkylgruppe sind Methyl, Ethyl, n-Propyl, iso-Propyl, n-Butyl, iso-Butyl, t-Butyl, etc.

Cycloalkyl steht insbesondere für C₃-C₆-Cycloalkyl, wie Cyclopropyl, Cyclobutyl, Cyclopentyl und Cyclohexyl.

Alkylen steht für geradkettige oder verzweigte Reste. Wenn A keine Gruppe Z aufweist, umfasst A 4 bis 10 Kohlenstoffatome, bevorzugt 4 bis 8 Kohlenstoffatome. Die Kette zwischen Triazolkern und Gruppe B weist dann mindestens vier Kohlenstoffatome auf. Wenn A wenigstens eine der genannten Gruppen Z aufweist, umfasst A 3 bis 10 Kohlenstoffatome, vorzugsweise 3 bis 8 Kohlenstoffatome.

Wenn die Alkylengruppen wenigstens eine der Gruppen Z umfassen, können diese in der Alkylenkette an beliebiger Stelle oder in Position 1 oder 2 der Gruppe A (vom Rest Ar¹ her gesehen) angeordnet sein. Die Reste CONR² und COO sind vorzugsweise so angeordnet, dass jeweils die Carbonylgruppe dem Triazolring zugewandt ist. Besonders bevorzugt sind Verbindungen der Formel I, worin A für -Z-C₃-C₆-Alkylen, insbesondere -Z-CH₂CH₂CH₂-, -Z-CH₂CH₂CH₂CH₂-, -Z-CH₂CH=CHCH₂-, -Z-CH₂C(CH₃)=CHCH₂-, -Z-CH₂C(=CH₂)CH₂-, -Z-CH₂CH(CH₃)CH₂- oder für einen linearen -Z-C₇-C₁₀-Alkylenrest steht, wobei Z an den Triazolring gebunden ist. Z steht vorzugsweise für CH₂, O und insbesondere S. Weiterhin bevorzugt steht A für -(CH₂)₄-, -(CH₂)₅-, -CH₂CH₂CH=CHCH₂-, -CH₂CH₂C(CH₃)=CHCH₂-, -CH₂C(=CH₂)CH₂- oder -CH₂CH₂CH(CH₃)CH₂-.

Halogen bedeutet F, Cl, Br oder I.

Halogenalkyl kann ein oder mehrere, insbesondere 1, 2, 3 oder 4 Halogenatome umfassen, die sich an einem oder mehreren C-Atomen befinden können, vorzugsweise in α- oder ω-Position. Besonders bevorzugt sind CF₃, CHF₂, CF₂Cl oder CH₂F.

Acyl steht vorzugsweise für HCO oder C₁-C₆-Alkyl-CO, insbesondere Acetyl.
Wenn Ar¹ substituiert ist, kann sich der Substituent auch an dem Stickstoffheteroatom befinden.

Vorzugsweise steht Ar¹ für 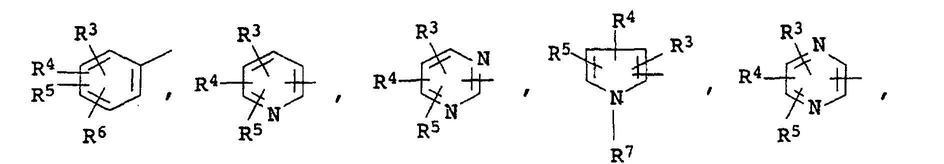 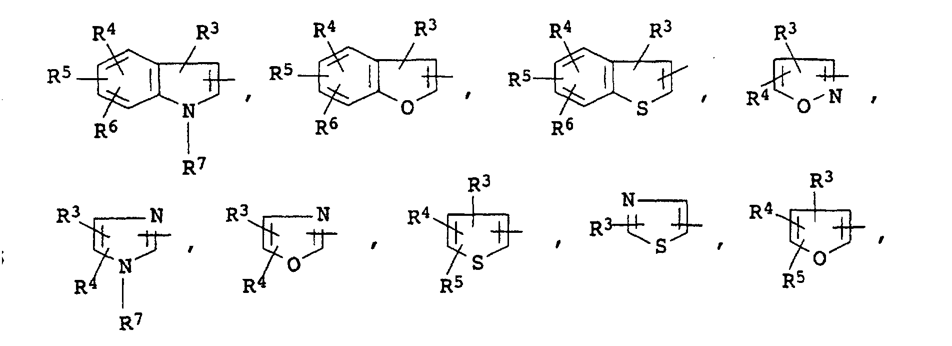 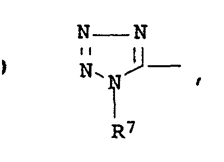 worin
R³ bis R⁶ für H oder die oben genannten Substituenten des Restes Ar¹ stehen,
R⁷ für H, C₁-C₆-Alkyl oder Phenyl steht und
X für N oder CH steht. Wenn der Phenylrest substituiert ist, stehen die Substituenten vorzugsweise in m- oder p-Stellung.

Besonders bevorzugt steht Ar¹ für 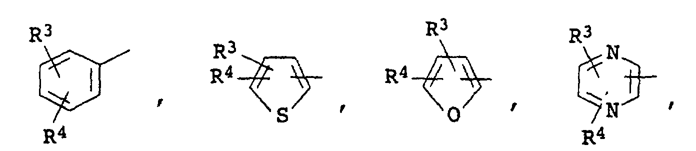 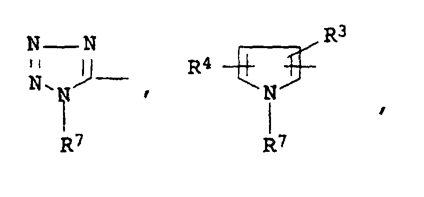 worin R³, R⁴ und R⁷ die oben angegebenen Bedeutungen besitzen. Die angegebenen Phenyl-, Pyrazinyl- und Pyrrolreste sind insbesondere bevorzugt.

Die Reste R³ bis R⁶ stehen vorzugsweise für H, C₁-C₆-Alkyl, OR², CN, Phenyl, das gegebenenfalls durch C₁-C₆-Alkyl, C₁-C₆-Alkoxy oder Halogen substituiert ist, CF₃ und Halogen und insbesondere für H, C₁-C₆-Alkyl, OR² und Halogen. R² besitzt dabei die oben angegebenen Bedeutungen.

Der Rest B steht vorzugsweise für 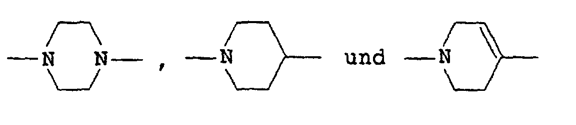 und insbesondere für

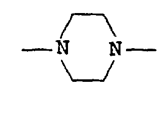

Der Rest Ar² kann einen, zwei, drei oder vier Substituenten, vorzugsweise einen oder zwei Substituenten, die sich insbesondere in m-Stellung und/oder p-Stellung befinden, aufweisen. Vorzugsweise sind sie unabhängig voneinander ausgewählt unter C₁-C₆-Alkyl, Halogenalkyl, NO₂, Halogen, insbesondere Chlor, Phenyl, Pyrrolyl, Imidazolyl, Pyrazolyl, Thienyl, Cyclopentyl und Cyclohexyl. Wenn einer der Substituenten für C₁-C₆-Alkyl steht, ist eine verzweigte Gruppe und insbesondere Isopropyl oder t-Butyl bevorzugt.

Vorzugsweise steht Ar² für gegebenenfalls substituiertes Phenyl, 2-, 3- oder 4-Pyridinyl oder 2-, 4(6)- oder 5- Pyrimidinyl.

Wenn einer der Substituenten des Restes Ar² für einen 5- oder 6-gliedrigen heterocyclischen Ring steht, so handelt es sich beispielsweise um einen Pyrrolidin-, Piperidin-, Morpholin-, Pyridin-, Pyrimidin-, Triazin-, Pyrrol-, Thiophen- oder Pyrazolrest, wobei ein Pyrrol-, Pyrrolidin-, Pyrazol- oder Thienylrest bevorzugt ist.

Wenn einer der Substituenten des Restes Ar² für einen carbocyclischen Rest steht, handelt es sich insbesondere um einen Phenyl-, Cyclopentyl- oder Cyclohexylrest.

Wenn Ar² mit einem carbocyclischen Rest kondensiert ist, handelt es sich insbesondere um einen Naphthalin-, Di- oder Tetrahydronaphthalinrest.

Gemäß einer Ausführungsform betrifft die Erfindung Verbindungen der Formel I, worin Ar¹ einen wie oben definierten heterocyclischen aromatischen Ring bedeutet, B für 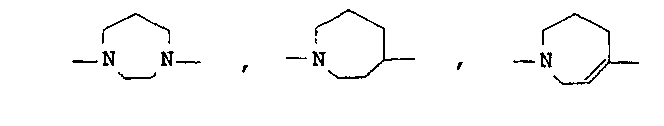 oder 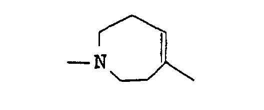 steht und A und Ar² die oben angegebenen Bedeutungen besitzen.

Die Erfindung umfaßt auch die Säureadditionssalze der Verbindungen der Formel I, mit physiologisch verträglichen Säuren. Als physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere brauchbare Säuren sind in Fortschritte der Arzneimittelforschung, Band 10, Seiten 224 ff., Birkhäuser Verlag, Basel und Stuttgart, 1966, beschrieben.

Die Verbindungen der Formel I können ein oder mehrere Asymmetriezentren aufweisen. Zur Erfindung zählen daher nicht nur die Racemate, sondern auch die betreffenden Enantiomere und Diastereomere. Auch die jeweiligen tautomeren Formen zählen zur Erfindung.

Das Verfahren zur Herstellung der Verbindungen (I) besteht darin, daß man
a) eine Verbindung der allgemeinen Formel (II) 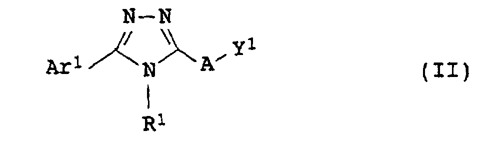 worin Y¹ für eine übliche Abgangsgruppe wie beispielsweise Hal, Alkansulfonyloxy, Arylsulfonyloxy etc. steht, mit einer Verbindung der allgemeinen Formel (III)

   HB-Ar² (III)

   umsetzt; oder
b) eine Verbindung der allgemeinen Formel (IV) 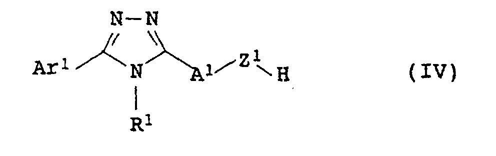 worin Z¹ für O, NR², oder S und A¹ für C₁-C₁₀-Alkylen oder eine Bindung steht, mit einer Verbindung der allgemeinen Formel (V)

   Y¹ - A² - B - Ar² (V)

   wobei Y¹ die oben angegebene Bedeutung besitzt und A² für C₂-C₁₀-Alkylen steht, wobei A¹ und A² zusammen 3 bis 10 C-Atome aufweisen und A¹ und/oder A² gegebenenfalls wenigstens eine Gruppe Z umfassen, umsetzt; oder
c) eine Verbindung der allgemeinen Formel (VI) 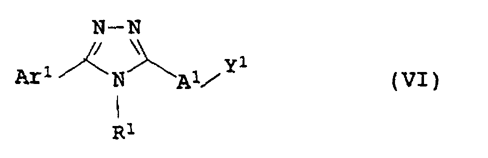 worin Y¹ und A¹ die oben angegebenen Bedeutungen besitzen, mit einer Verbindung der allgemeinen Formel (VII)

   H - Z¹ - A - B - Ar² (VII)

   worin Z¹ die oben angegebenen Bedeutungen besitzt, umsetzt; oder
d) eine Verbindung der allgemeinen Formel (VIII) 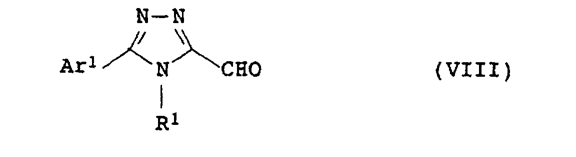 mit literaturbekannten Reagenzien, wie z. B. 1,3-Propandithiol, KCN/Wasser, TMSCN oder KCN/Morpholin, wie z. B. beschrieben in
   Albright *Tetrahedron,* **1983,** 39, 3207 oder
   D. Seebach *Synthesis* **1969,** 17 und **1979,** 19 oder
   H. Stetter *Angew. Chem. Int. Ed.* **1976,** 15, 639 oder
   van Niel et al. *Tetrahedron* **1989,** 45, 7643
   Martin et al. *Synthesis* **1979,** 633,
   zu den Produkten (VIIIa) (exemplarisch mit 1,3-Propandithiol) 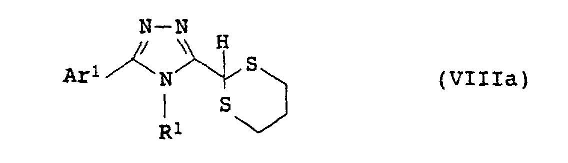 umpolt und anschließend mit Verbindungen der allgemeinen Formel (IX)

   Y¹ - A³ - B - Ar² (IX)

   wobei Y¹ die oben angegebene Bedeutung besitzt und A³ für C₃-C₉-Alkylen steht, das eine Gruppe Z enthalten kann, kettenverlängert, wobei man nach Entschützen oder Reduktion
   Verbindungen der Formel (Ia) 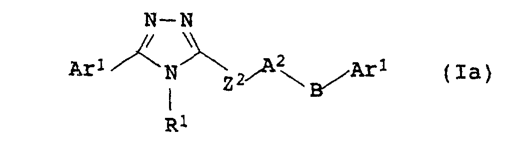 worin Z² für CO oder eine Methylengruppe steht und Z² und A² zusammen 4 bis 10 C-Atome aufweisen, erhält, oder
e) eine Verbindung der allgemeinen Formel (VIII) mit einer Verbindung der allgemeinen Formel X

   Y² - A - B - Ar² (X)
worin Y² für ein Phosphoran oder einen Phosphonsäureester steht, analog nach üblichen Methoden, wie zum Beispiel beschrieben in Houben Weyl *"Handbuch der Organischen Chemie"* 4. Auflage, Thieme Verlag Stuttgart, Band V/1b S.383 ff oder Bd V/1c S.575 ff, umsetzt.

Das Verfahren zur Herstellung einer Verbindung der Formel I, worin A die Gruppe COO oder CONR² umfasst, besteht darin, daß man eine Verbindung der allgemeinen Formel (XI) 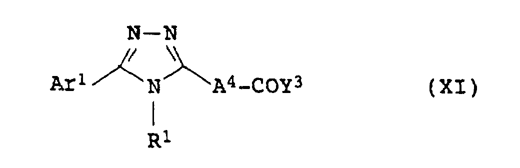 worin Y³ für OH, OC₁-C₄, Cl oder zusammen mit CO für eine aktivierte Carboxylgruppe, und A⁴ für C₀-C₉-Alkylen steht, mit einer Verbindung der Formel (XII)

Z³- A - B - Ar² (XII)

worin Z³ für OH und NHR² steht, umsetzt.

Die Verbindungen der Formel (III) sind Ausgangsverbindungen zur Herstellung von Verbindungen der Formeln (V), (VII) und (XII) und werden hergestellt durch Standardmethoden, wie z. B. beschrieben in J.A. Kiristy et al., *J. Med. Chem.* **1978,** *21,* 1303 oder C.B. Pollard, *J. Am. Chem. Soc.* **1934,** *56,* 2199, oder indem man
a) eine Verbindung der allgemeinen Formel (XIII) 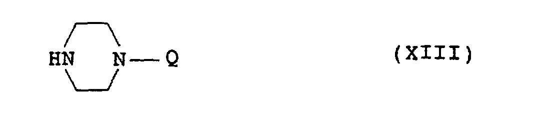 worin Q für H oder eine übliche Aminoschutzgruppe steht, mit einer Verbindung der allgemeinen Formel (XIV)

   Y⁴ - Ar² (XIV)

   worin Y⁴ für B(OH)₂, -SnR₃ (R₃ = Butyl oder Phenyl), Trifluormethansulfonyloxy steht oder die für Y¹ angegebenen Bedeutungen besitzt und R für C₁-C₄-Alkyl steht, in bekannter Weise umsetzt; oder
b) eine Verbindung der allgemeinen Formel (XV)

   Q - B¹ (XV)

   worin B¹ für 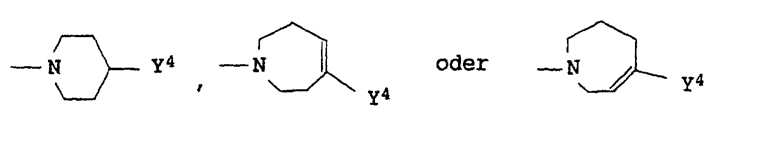 steht, Q für H oder eine übliche Aminoschutzgruppe, z. B. Butyloxycarbonyl, Benzyl oder Methyl, steht und Y⁴ für eine Abgangsgruppe, z. B. OTf, SnBu₃, B(OH)₂ oder Halogen, steht, mit einer Verbindung der allgemeinen Formel (XIVa)

   Y⁵ - Ar² (XIVa)

   worin Y⁵ für Borderivate, wie z. B. B(OH)₂ oder eine metallhaltige Abgangsgruppe, z. B. SnR₃ (R₃ = Butyl oder Phenyl) oder Zinkhalogenid steht, wenn Y⁴ für Halogen oder Trifluormethylsulfonyloxy steht oder Y⁵ für Halogen oder Trifluormethylsulfonyloxy steht, wenn Y⁴ für Borderivate, wie z. B. B(OH)₂ oder eine metallhaltige Abgangsgruppe, z. B. SnR₃ oder Zinkhalogenide, steht, nach bekannten Verfahren umgesetzt, wie beschrieben in
   S. Buchwald et al. *Angew. Chem.* **1995,** *107*, 1456 oder J.F.
   Hartwig et al. *J. Am. Chem. Soc.* **1996,** *118*, 7217 oder
   S. Buchwald *J. Org. Chem.* **1997,** *62,* 1264 oder F. Kerrigan et al., *Tetrah. Lett.* **1998,** *39,* 2219 und dort zitierte Literatur oder
   J.K. Stille, *Angew. Chem.* **1986,** *98,* 504 oder
   J.K. Stille et al., *J. Org. Chem*. **1990,** *55*, 3014. M. Pereyre et al. "Tin in Organic Synthesis", Butterworth 1987; oder
c) eine Verbindung der allgemeinen Formel (XVI) 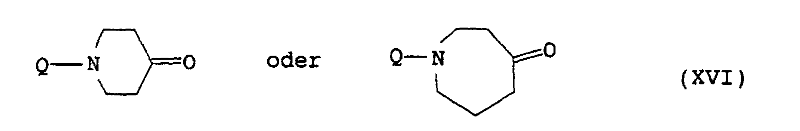 worin Q die oben angegebene Bedeutung besitzt, mit einer Verbindung M-Ar², worin M für ein Metall wie z. B. Li, MgY⁶ , und Y⁶ für Br, Cl, I steht, umsetzt. M-Ar² kann nach literaturbekannten Methoden aus Verbindungen der Formel (XIV) erhalten werden, oder
d) eine Verbindung der allgemeinen Formel (XVII)

   Q-B²-Ar² (XVII)

   worin B² für 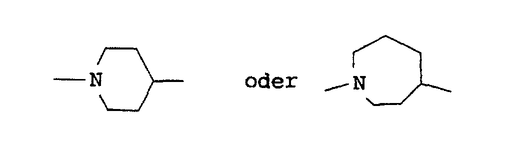 steht und Q die oben angegebene Bedeutung besitzt, durch Reduktion, z. B. Hydrierung, von Verbindungen der allgemeinen Formel Q-B³-Ar² (IIIa), worin B³ für einen der oben genannten ungesättigten Reste B steht, in literaturbekannter Weise herstellt.

Verbindungen des Typs B sind entweder bekannt oder sie können analog zu bekannten Verfahren hergestellt werden, wie z. B. 1,4-Diazacycloalkane: L. Borjeson et al., Acta Chem. Scand. 1991, 45, 621; Majahrzahl et al Acta Pol. Pharm., 1975, 32, 145, 1-Azacycloheptanone: A. Yokoo et al., Bull Chem. Soc. Jpn. 1956, 29, 631 und WO 97/25324.

In obigen Formeln besitzen Ar¹, R¹, A, B, Z und Ar² die oben angegebenen Bedeutungen.

Verbindungen des Typs Ar¹-Triazol, Ar², Ar¹ sind entweder bekannt oder können nach bekannten Verfahren hergestellt werden wie z.B. beschrieben in S. Kubota et al. *Chem. Pharm. Bull* 1975, *23*, 955 oder A.R. Katritzky, C.W. Rees(ed.) "Comprehensive Heterocyclic Chemistry", Pergamon Press, oder "The Chemistry of Heterocyclic Compounds"' J. wiley & Sons Inc. NY und der dort zitierten Literatur.

Die Verbindungen der Formel VIII sind neu und ebenfalls Gegenstand der vorliegenden Erfindung.

Verbindungen des Typs (VIII) und (XI), wobei A für C₀-Alkylen steht, können durch Metallierung der 3-Aryl-5-H-1,2,4(4H)-Triazole 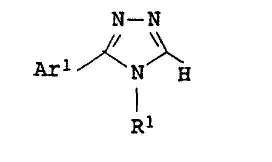 und analog den bei T. Kauffman et al. Angew. Chem. Int. Ed. Engl. **1972**, *11*, 846 oder von A.R. Katritzky, C.W. Rees(ed.) "Comprehensive Heterocyclic Chemistry", Pergamon Press Vol 5, p 753 beschriebenen Methoden hergestellt werden.

Die Herstellung der erfindungsgemäßen Verbindungen und der Ausgangsmaterialien und der Zwischenprodukte kann auch analog zu den in den eingangs genannten Patentpublikationen beschriebenen Methoden erfolgen.

Die oben beschriebenen Umsetzungen erfolgen im allgemeinen in einem Lösungsmittel bei Temperaturen zwischen Raumtemperatur und der Siedetemperatur des verwendeten Lösungsmittels. Brauchbare Lösungsmittel sind beispielsweise Ester, wie Ethylacetat, Ether, wie Diethylether oder Tetrahydrofuran, Dimethylformamid, Dimethylsulfoxid, Dimethoxyethan, Toluol, Xylol, Ketone, wie Aceton oder Methylethylketon, oder Alkohole, wie Ethanol oder Butanol.

Gewünschtenfalls arbeitet man in Gegenwart eines säurebindenden Mittels. Geeignete säurebindende Mittel sind anorganische Basen, wie Natrium- oder Kaliumcarbonat, Natrium- oder Kaliumhydrogencarbonat, Natriumethylat, Natriumhydrid oder metallorganische Verbindungen, wie Butyllithium- oder Alkylmagnesium-Verbindungen, oder organische Basen, wie Triethylamin oder Pyridin. Letztere können gleichzeitig als Lösungsmittel dienen.

Die Umsetzungen erfolgen gegebenenfalls unter Verwendung eines Katalysators, wie z.B. Übergangsmetalle und deren Komplexe, z.B. Pd(PPh₃)₄, Pd(OAc)₂ oder Pd(P(oTol)₃)₄, oder eines Phasen-Transfer-Katalysators, z.B. Tetrabutylammoniumchlorid oder Tetrapropylammoniumbromid.

Die Isolierung des Rohprodukts erfolgt in üblicher Weise, beispielsweise durch Filtration, Abdestillieren des Lösungsmittels oder Extraktion aus dem Reaktionsgemisch etc. Die Reinigung der erhaltenen Verbindungen kann in üblicher Weise erfolgen, beispielsweise durch Umkristallisieren aus einem Lösungsmittel, Chromatographie oder Überführen in eine Säureadditionsverbindung.

Die Säureadditionssalze werden in üblicher Weise durch Mischen der freien Base mit der entsprechenden Säure, gegebenenfalls in Lösung in einem organischen Lösungsmittel, beispielsweise einem niedrigen Alkohol, wie Methanol, Ethanol oder Propanol, einem Ether, wie Methyl-t-butylether, einem Keton, wie Aceton oder Methylethylketon oder einem Ester, wie Essigsäureethylester, hergestellt.

Zur Behandlung der oben erwähnten Erkrankungen werden die erfindungsgemäßen Verbindungen in üblicher Weise oral oder parenteral (subkutan, intravenös, intramuskulär, intraperitoneal) verabreicht. Die Applikation kann auch mit Dämpfen oder Sprays durch den Nasen-Rachen-Raum erfolgen.

Die Dosierung hängt vom Alter, Zustand und Gewicht des Patienten sowie von der Applikationsart ab. In der Regel beträgt die tägliche Wirkstoffdosis etwa 10 bis 1000 mg pro Patient und Tag bei oraler Gabe und etwa 1 bis 500 mg pro Patient und Tag bei parenteraler Gabe.

Die Erfindung betrifft auch pharmazeutische Mittel, die die erfindungsgemäßen Verbindungen enthalten. Diese Mittel liegen in den üblichen galenischen Applikationsformen in fester oder flüssiger Form vor, beispielsweise als Tabletten, Filmtabletten, Kapseln, Pulver, Granulate, Dragees, Suppositorien, Lösungen oder Sprays. Die Wirkstoffe können dabei mit den üblichen galenischen Hilfsmitteln, wie Tablettenbindemitteln, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al., Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die nachfolgenden Beispiele dienen zur Erläuterung der Erfindung ohne sie zu begrenzen.

### BEISPIEL 1

### 3-{3-[4-(2-t-Butyl-6-trifluormethyl-pyrimidin-4-yl-)-piperazin-1-yl]-propylmercapto}-4-methyl-5-phenyl-1,2,4-(4H)-triazol

### A. Herstellung der Ausgangsverbindungen:

A.1 2-t-Butyl-4-[4-(3-chlorpropyl)-piperazin-1-yl]-6-trifluormethylpyrimidin und 2,2-Dimethylpropanimidamid wurden in bekannter Weise mit Trifluoressigsäureethylester zu 2-(2,2-Dimethylethyl)-4-hydroxy-6-trifluormethylpyrimidin umgesetzt. Heterocyclic Compounds (John Wiley & Sons, 1994, Vol. 52, D.J. Brown (Hrsg.).
   C₉H₁₁F₃N₂O Fp. 187-188° C.
A.2 Nach Chlorierung mit Thionylchlorid wurde das Rohprodukt mit einem Überschuß an wasserfreiem Piperazin behandelt, wobei 2-t-Butyl-4-piperazin-1-yl-6-trifluormethylpyrimidin erhalten wurde.
   C₁₃H₁₉F₃N₄ Fp. 78-80° C.
A.3 Nach Alkylierung der erhaltenen Verbindung in Tetrahydrofuran mit 1-Brom-3-chlorpropan wurde 2-t-Butyl-4-[4-(2-chlorpropyl)-piperazin-1-yl]-6-trifluormethyl-pyrimidin erhalten.
   C₁₆H₂₄ClF₃N₄ Fp. 83-84° C.
   Die eingesetzten Triazole wurden, soweit nicht anders angegeben, nach der Methode von S. Kubota et al, Chem Pharm Bull. **1975**,*23*,955 durch Umsetzung der entsprechenden Carbonsäurechloride mit Alkylthiosemicarbaziden in Pyridin und anschließender zyklisierung in wäßriger Natriumhydrogencarbonat-Lösung bzw. Addition der entsprechenden Carbonsäurehydrazide mit Alkyisothiocyanaten in einem geeigneten Lösungsmittel, hergestellt.
A.4 4-Methyl-3-mercapto-5-(thiophen-3-yl)-1,2,4-(4H)-triazol Es wurde das Natriumsalz isoliert.
   1H-NMR (DMSO-d₆): 3.7 (3H); 7.5 (m, 2H); 7.8 (m,1H).
   Smp: 146°C
   C₇H₆N₃S₂Na (219)
A.5 4-Methyl-3-mercapto-5-(2,5-dimethyl-furan-3-yl)-1,2,4-(4H)-triazol
   1H-NMR (DMSO-d₆): δ = 2.3 (s, 3H); 2.5 (s,3H); 3.7 (s, 3H); 6.1 (s, 1H).
A.6 4-Methyl-3-mercapto-5-(2,6-dichlor-phenyl)-1,2,4-(4H)-triazol
   Es wurde das Natriumsalz isoliert.
   1H-NMR (DMSO-d₆): δ = 3.7 (s, 3H); 7.4 (dd,1H); 7.6 (d, 1H); 8.2 (d, 1H).
   Smp: 220-225°C
A.7 4-Methyl-3-mercapto-5-(4-methylsulfony-phenyl)-1,2,4-(4H)-triazol
   1H-NMR (DMSO-d₆): δ = 3.7 (s, 3H); 7.4 (dd,1H); 7.6 (d, 1H); 8.2 (d, 1H).
   Smp: 238-239°C
A.8 4-Methyl-3-mercapto-5-(3-brom-pyridyl-5)-1,2,4-(4H)-triazol
   Es wurde das Natriumsalz isoliert.
   1H-NMR (DMSO-d₆): δ = 3.7 (s, 3H); 8.2(m,1H); 8.9 (m,2H).
A.9 4-Methyl-3-mercapto-5-(pyrrol-2-yl)-1,2,4-(4H)-triazol
   1H-NMR (DMSO-d₆): δ = 3.7 (s, 3H); 6.2(m,1H); 6.8 (1,2H); 7.0 (m,1H); 11.8 (s,1H); 14.0 (s,1H).
   Smp: 200-201°C
A.10 4-Methyl-3-mercapto-5-(3-benzthienyl) 1,2,4-(4H)-triazol
   Es wurde das Natriumsalz isoliert.
   1H-NMR (DMSO-d₆): 3.8 (s, 3H); 7.5(m,2H); 8.0 (m,3H).
A.11 4-Methyl-3-mercapto-5-(4-methyl-thiazol-5-yl)-1,2,4-(4H)-triazol
   1H-NMR (DMSO-d₆): 2.4 (s,3H); 3.4 (s, 3H),9.2 (s,1H); 14.1 (s, 1H).
A.12 4-Methyl-3-mercapto-5-(6-chlor-biphenyl-2)-1,2,4-(4H)-triazol
   1H-NMR (DMSO-d₆): 3.8 (s, 3H), 7.6 (m,1H), 7.9 (m,1H);8.1 (m,3H); 8.4 (s,1H).
A.13 4-Methyl-3-mercapto-5-(2,4-dinitrophenyl-)-1,2,4-(4H)-triazol
   Smp: 250-251°C
   MS: m/z= 281[M⁺]
A.14 4-Methyl-3-mercapto-5-(4-CF₃-phenyl)-1,2,4-(4H)-triazol
   MS: m/z= 259[M⁺]
A.15 4-Propyl-3-mercapto-5-(2-methyloxazol-4-yl)-1,2,4-(4H)-triazol
   Es wurde das Kaliumsalz isoliert.
   Eine Lösung von 4,9 g (22,5 mmol) 2-Methyloxazol-4-carbonsäurehydrazid-bishydrochlorid (hergestellt durch Hydrazinolyse des entsprechenden Methylesters in methanolischer Lösung) in 60 ml Ethanol wurde nacheinander mit 6,22 g (95 mmol) Kaliumcarbonat und 2,4 ml (23 mmol) Propylisothiocyanat versetzt und 4 h zum Sieden erhitzt.
   Die entstandene Suspension wurde filtriert, eingeengt und der Rückstand (6.5 g) säulenchromatographisch gereinigt. (Kieselgel, Methylenchlorid-Methanol 96:4)
   Ausbeute: 2,3 g (39 % d. Th.)
   1H-NMR (CDCl₃): δ= 1.0 (t, 3H): 1.7 (m,2H); 2.6 (s,3H); 4.2 (sm,2H); 8.1 (s,1H); 12.6 (s,1H).
A.16 4-Propyl-3-mercapto-5-(2-amino-thiazol-4-yl)-1,2,4-(4H)-triazol
   Es wurde das Kaliumsalz isoliert.
   1H-NMR (DMSO-d₆): 0.8 (t,3H); 1.6 (m,2H); 3.4 (s, 2H); 4.3 (m,2H); 7.4 (S,1H); 13.8.
A.17 4-Methyl-3-mercapto-5-(5-methylimidazol-4-yl)-1,2,4-(4H)-triazol
   Es wurde das Kaliumsalz isoliert.
   1H-NMR (DMSO-d₆): 2.3 (s,3H); 3.4 (s,3H); 7.5 (S,1H).
A.18 4-Methyl-3-mercapto-5-(carboxamido)-1,2,4-(4H)-triazol
   1H-NMR (DMSO-d₆): 3.7 (s,3H); 7.95 (s,1H); 8.25 (s,1H); 14.2 (s,1H).
   MS: m/z= 158[M⁺]
A.19 4-Methyl-3-mercapto-5-(N-methylpyrrol-2-yl)-1,2,4-(4H)-triazol
   10,2 g (45,1 mmol) 2-Trichloracetoxy-N-methylpyrrol (dargestellt nach Rappoport et al., J. Org. Chem. **1972**, 37, 3618) in DMF wurden mit 10,6 g (101,1 mmol) 4-Methyl-3-thiosemicarbazid und katalytischen Mengen Dimethylaminopyridin versetzt und 18 h bei 90 °C erhitzt. Bei Raumtemperatur gab man 77 ml Wasser zu, säuerte mit 10%iger HCl an, rührte 1 h bei 0 °C, filtrierte vom Ungelösten ab und extrahierte die Mutterlauge mit Essigester. Die organischen Phasen wurden getrocknet, evaporiert und das erhaltene Rohprodukt mit 427 ml 1M Natriumhydrogencarbonat-Lösung zum Sieden erhitzt. Nach beendeter Reaktion filtrierte man vom Ungelösten ab, säuerte die Mutterlauge unter Kühlung mit konz. HCl an und isolierte den ausgefallenen Feststoff.
   Ausbeute: 2,3 g (27% d.Th)
   MS: m/z= 194 [M+]
   1H-NMR (DMSO-d₆): δ = 3.6 (s,3H); 3.9 (s,3H); 6.2 (m,1H); 6.6 (m,1H); 7.1 (m,1H); 14.0 (1H).

### B. Herstellung des Endproduktes:

576 mg (3 mmol) 4-Mercapto-3-methyl-5-phenyl-1,2,4-(4H)-triazol (hergestellt nach der Methode von S. Kubota u. M. Uda, Chem. Pharm. Bull. (1975), 23, 955-966 durch Umsetzung von Benzoylchlorid mit N-Methylthiosemicarbazid und nachfolgender Cyclisierung) und 1,1 g (3 mmol) der vorstehend unter A.3 beschriebenen Chlorpropylverbindung wurden in 10 ml trockenem DMF zusammen mit 7,2 mg (3 mmol) Lithiumhydroxid 6 h unter Rühren auf 100°C erwärmt. Nach dem Abkühlen wurde mit 50 ml Wasser versetzt und 3mal mit t-Butylmethylether extrahiert. Die organische Phase wurde mit Natriumsulfat getrocknet und eingedampft, der Rückstand säulenchromatographisch (Kieselgel) gereinigt. Die erhaltene reine Substanz (920 mg = 59 %) wurde anschließend mit etherischer Salzsäure in ihr Hydrochlorid umgewandelt.
C₂₅H₃₃ClF₃N₇S (556) Fp. 191-193°C.

In analoger weise wurden die folgenden in tabellarischer Form aufgeführten Substanzen der allgemeinen Formel (I) erhalten.

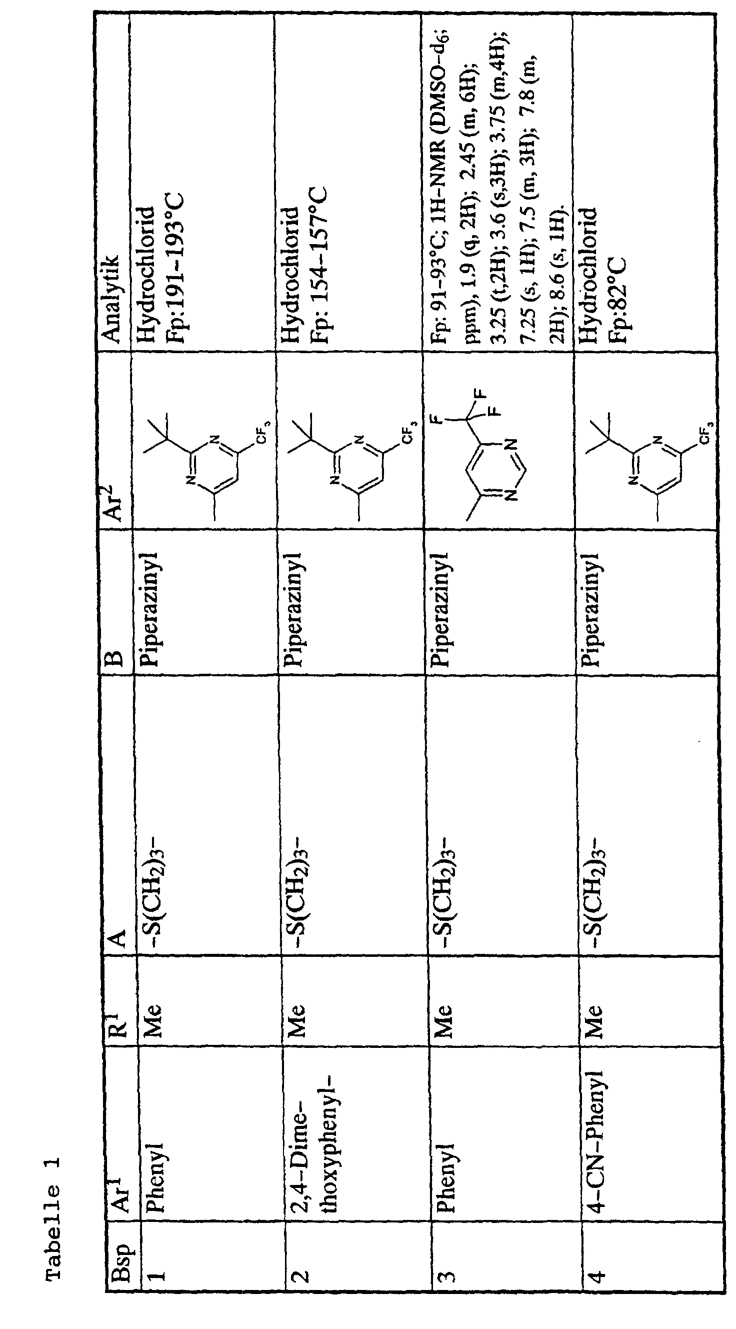

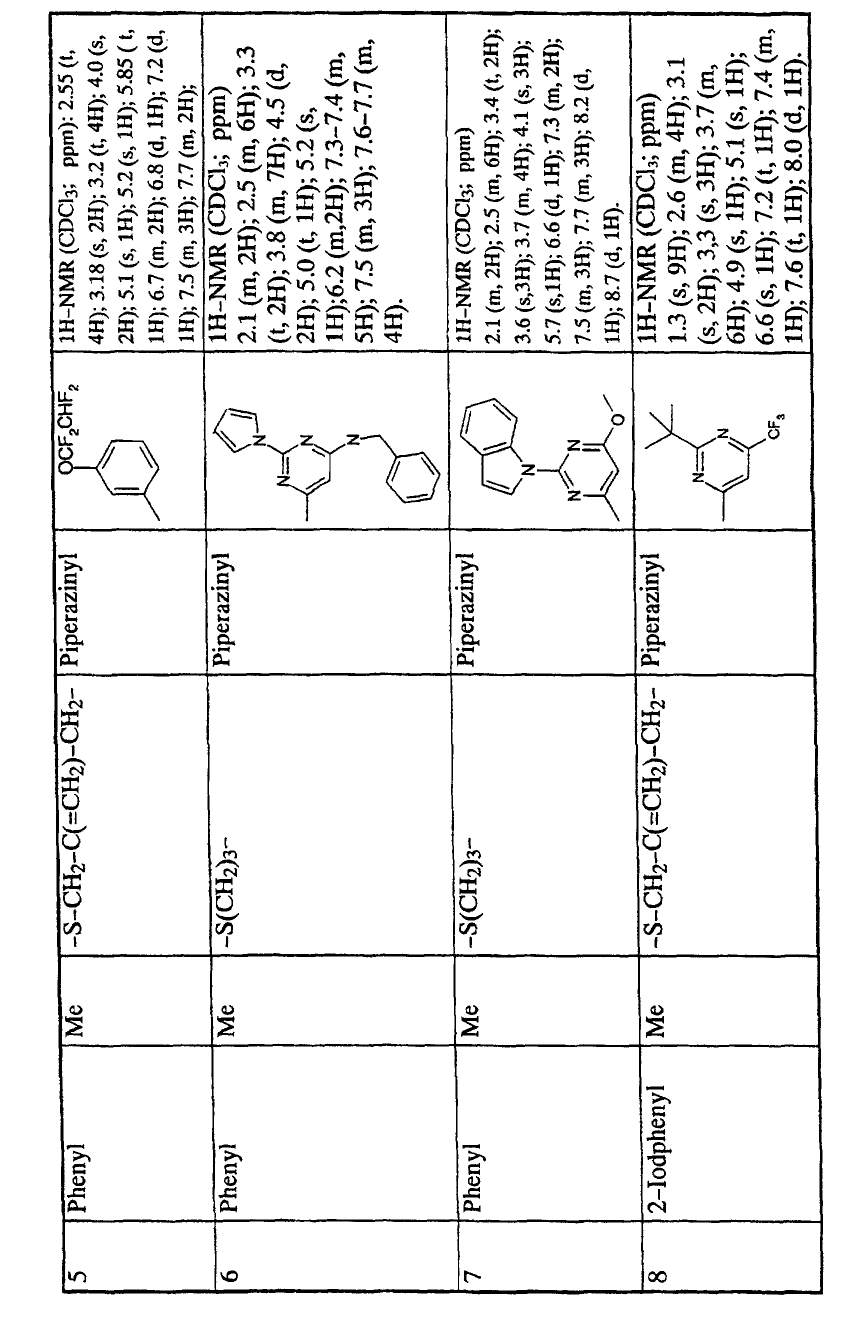

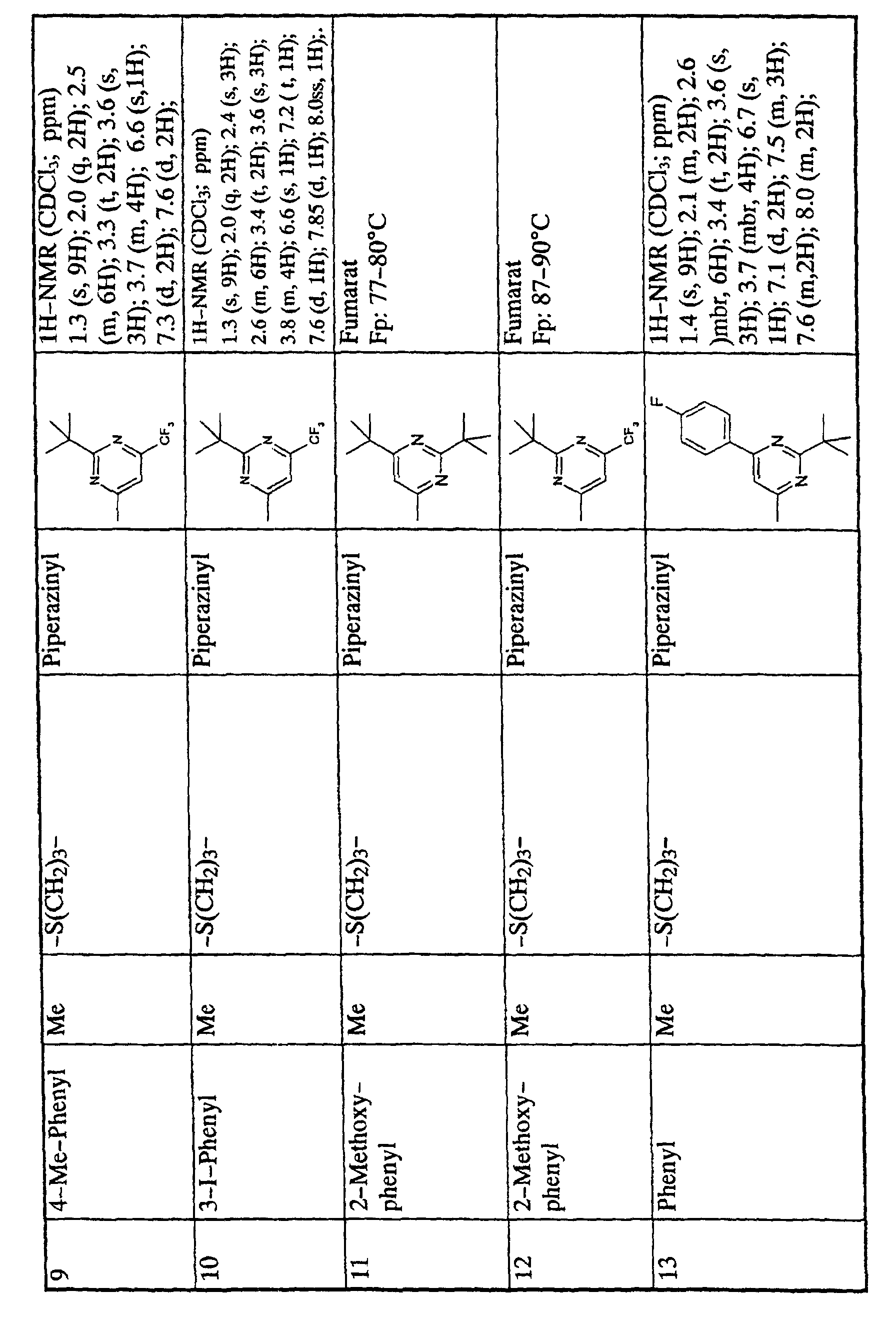

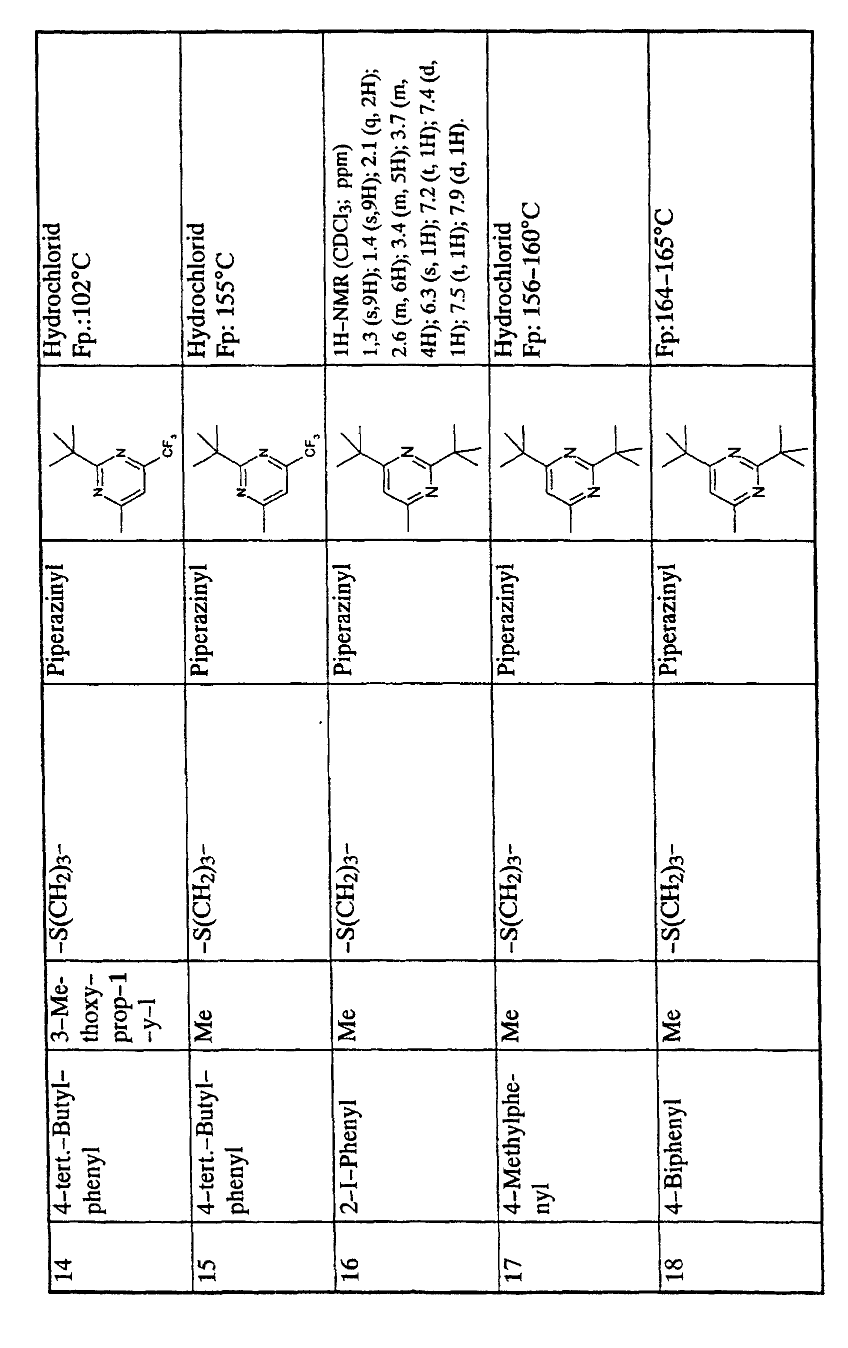

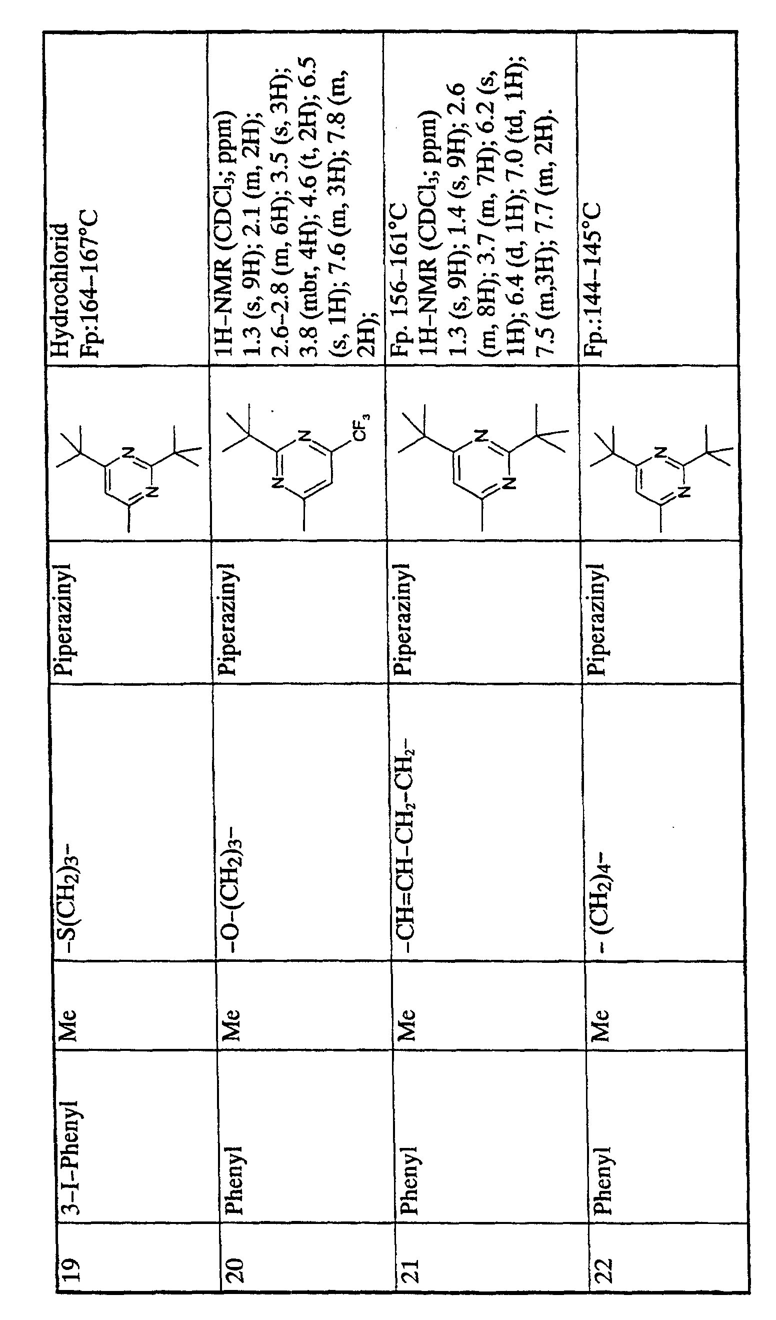

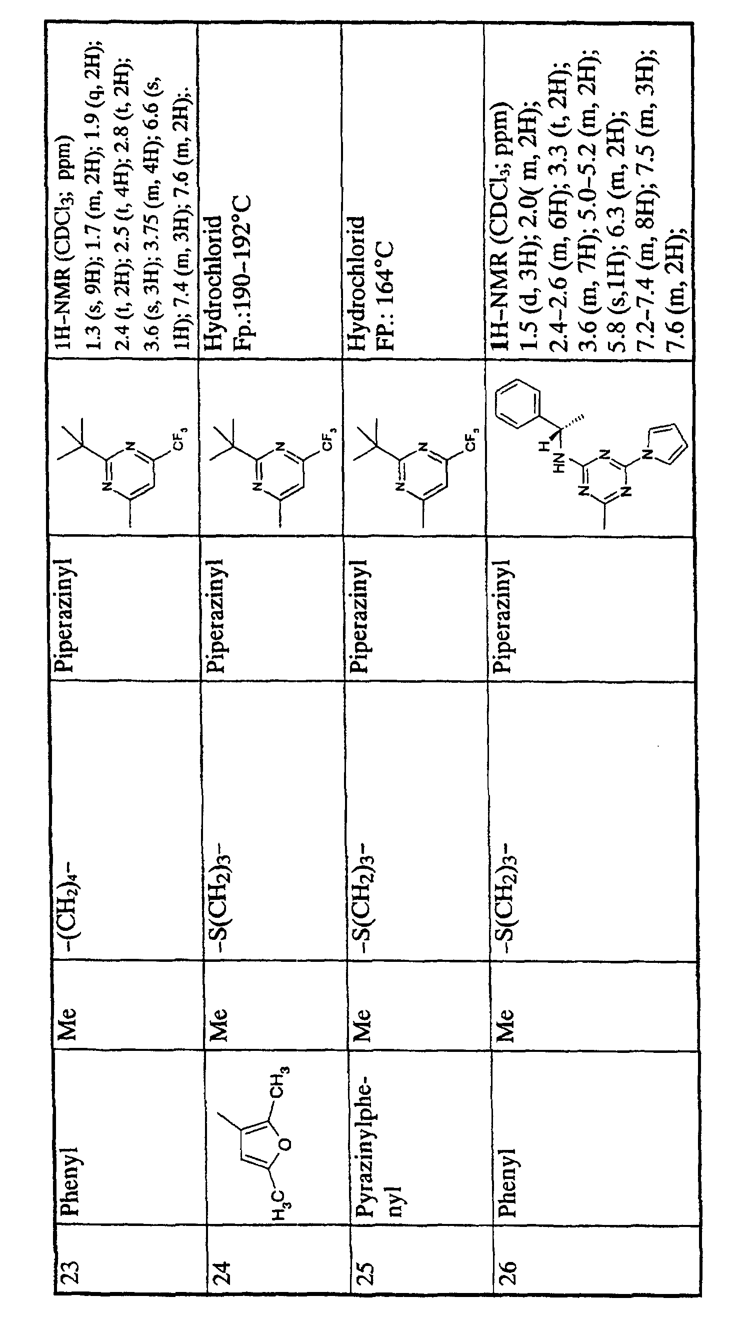

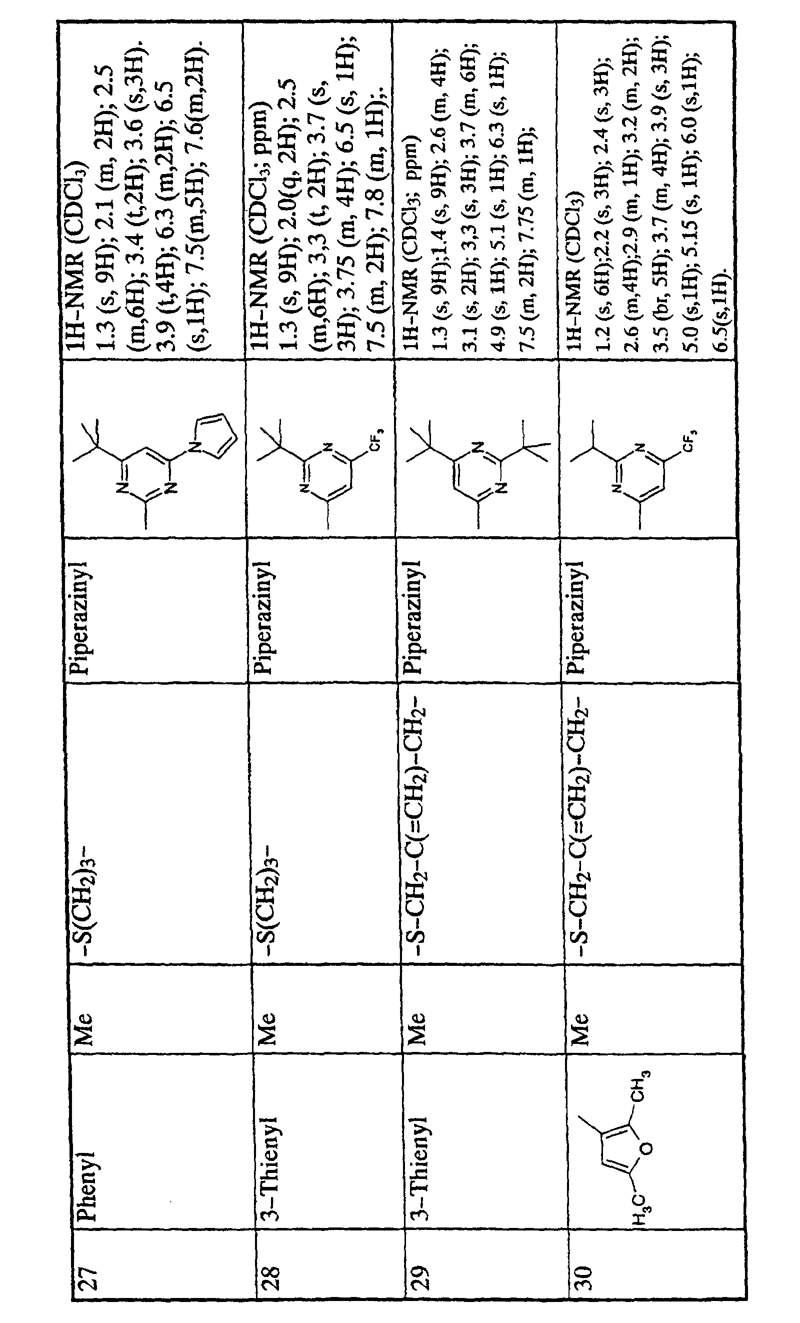

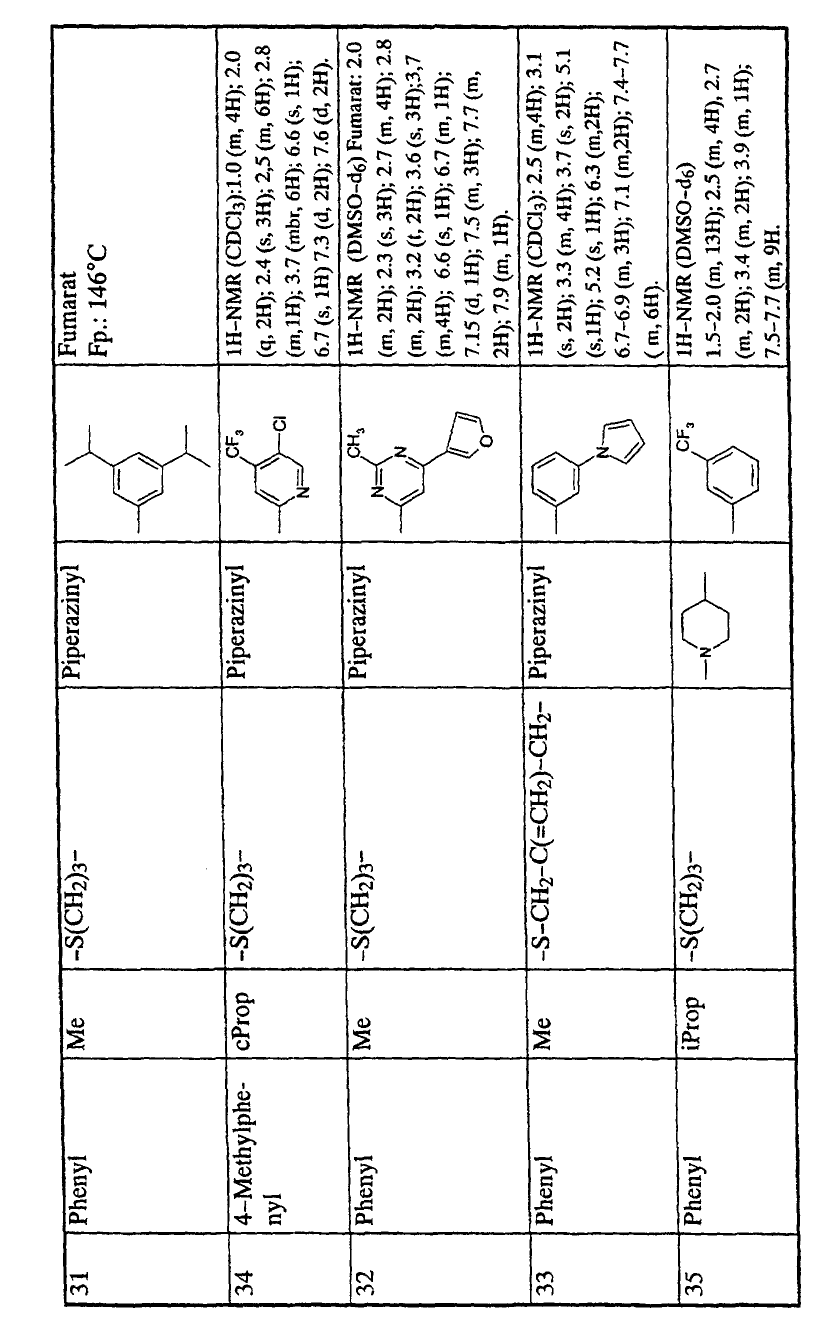

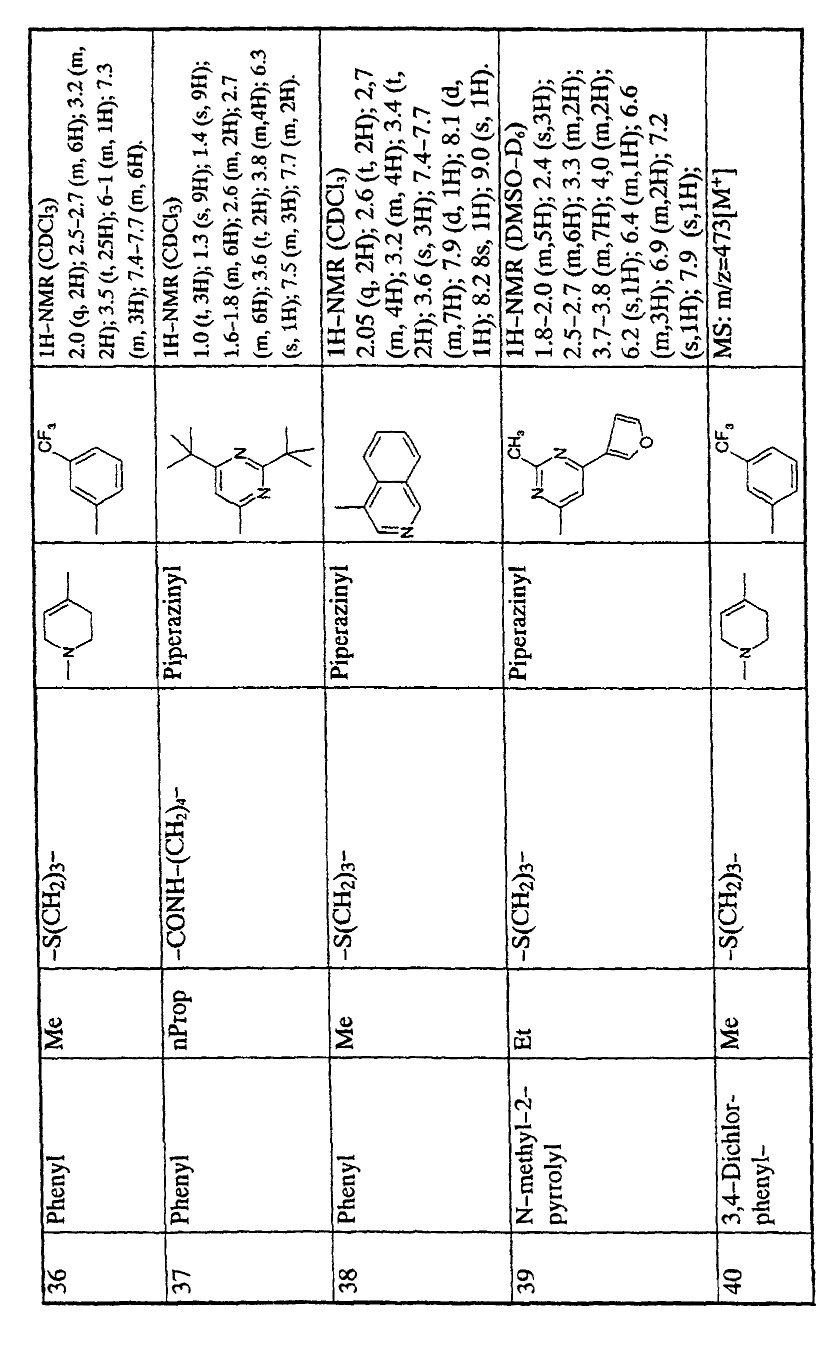

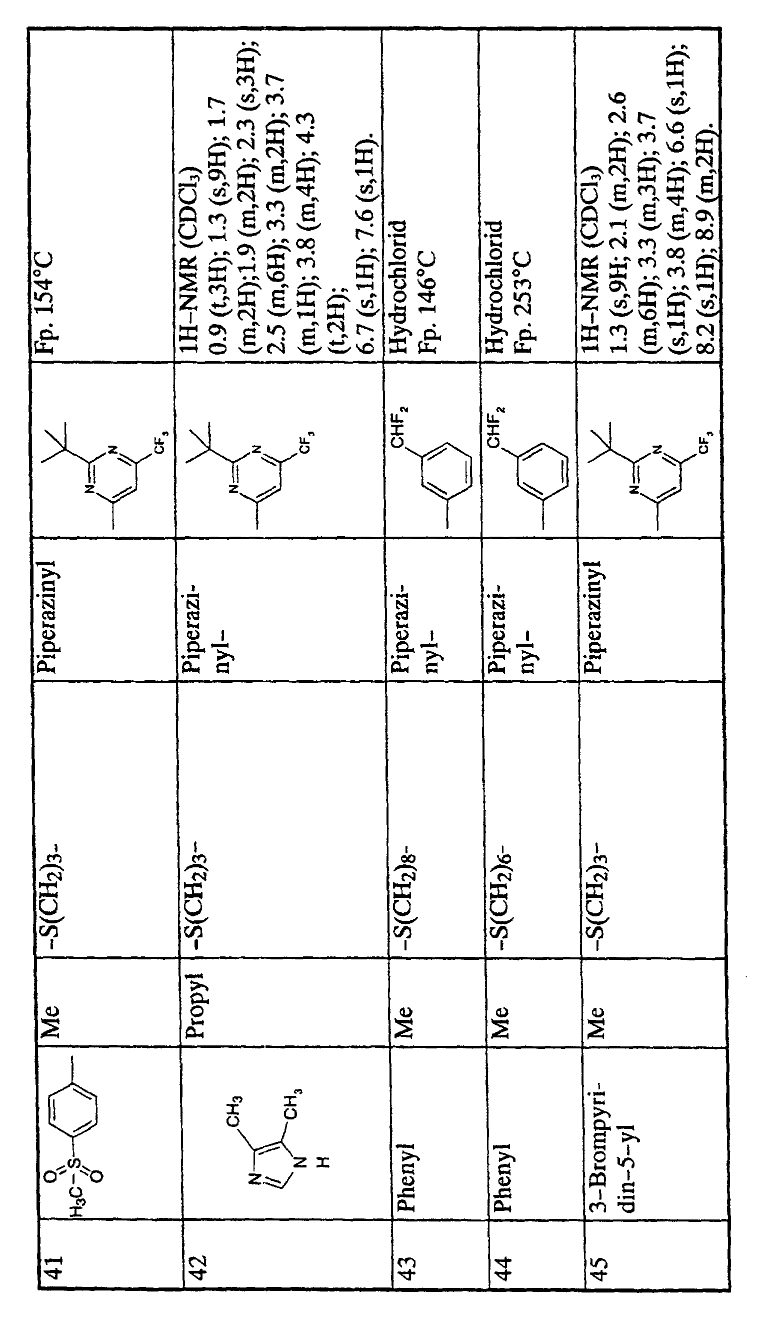

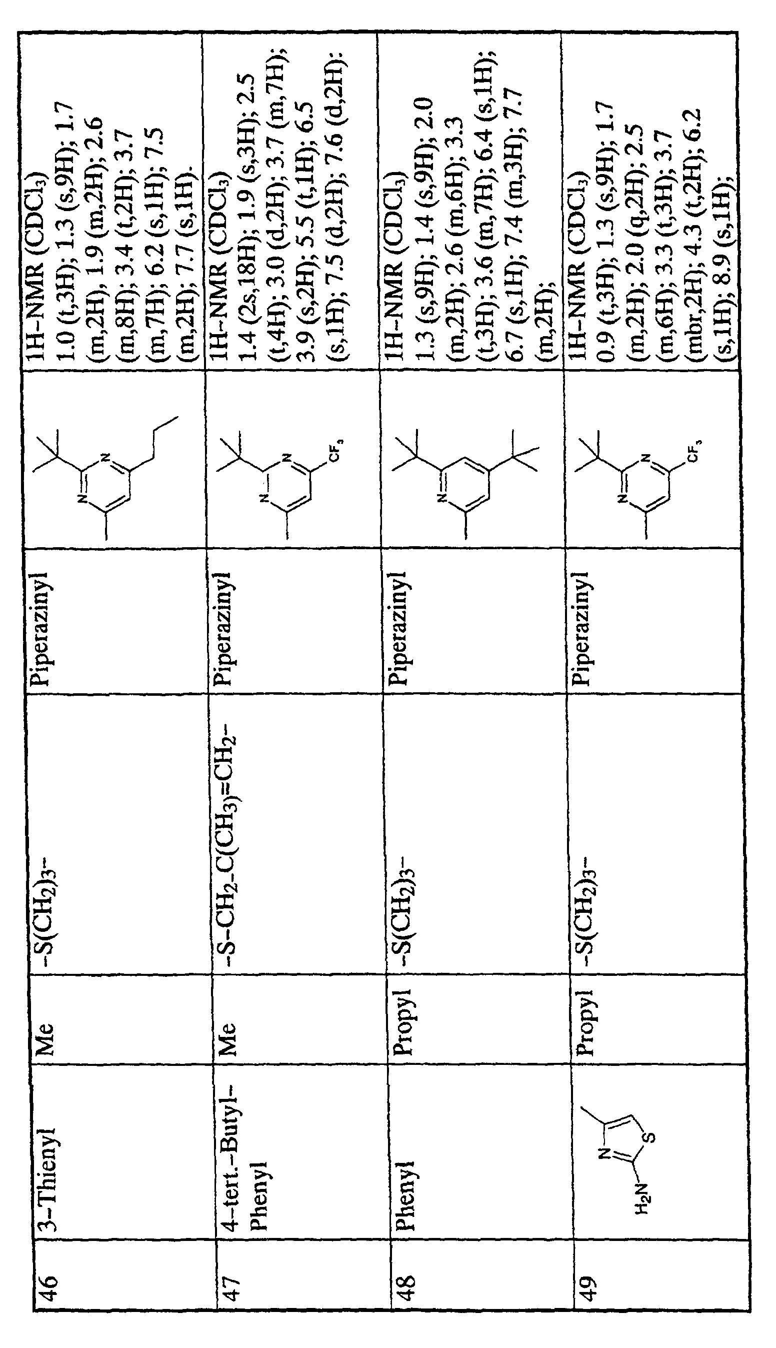

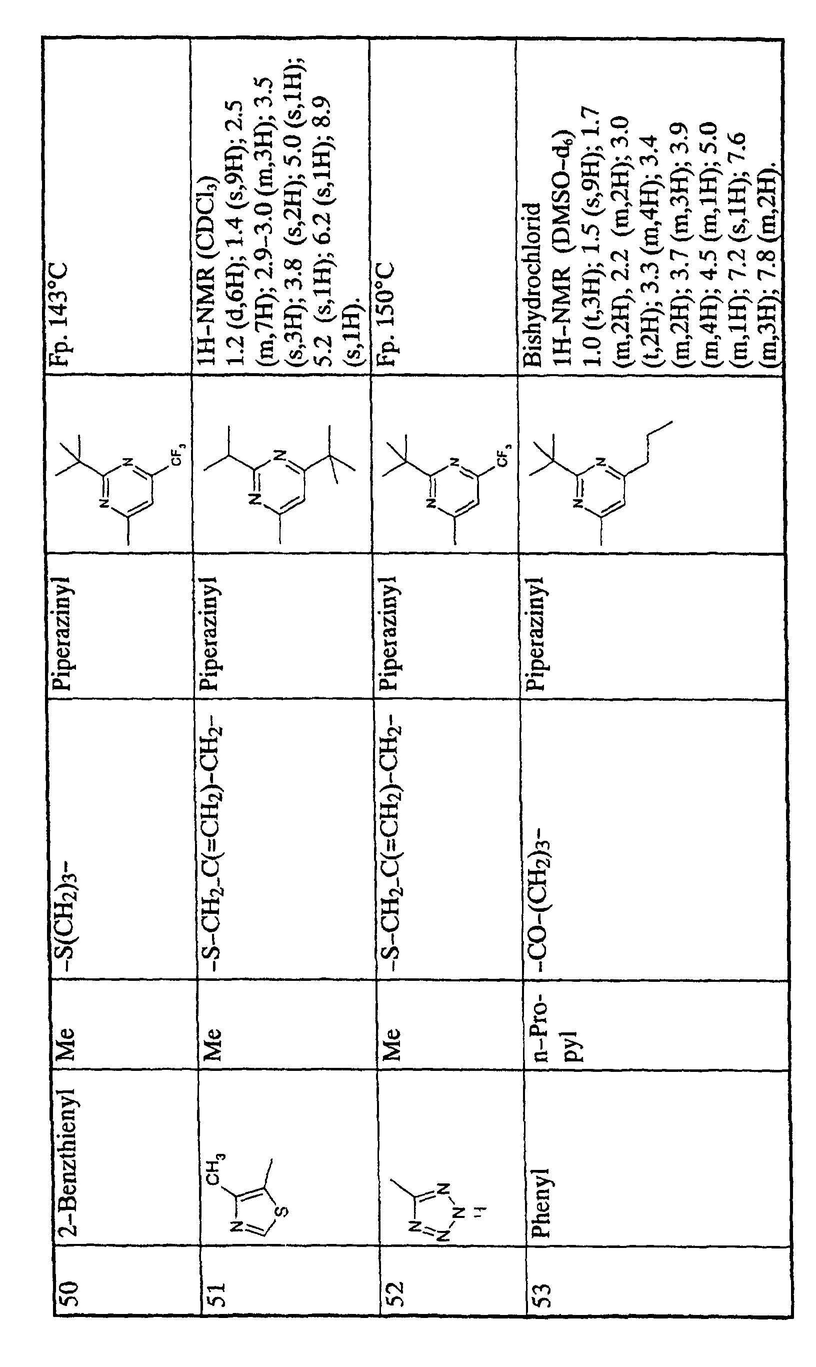

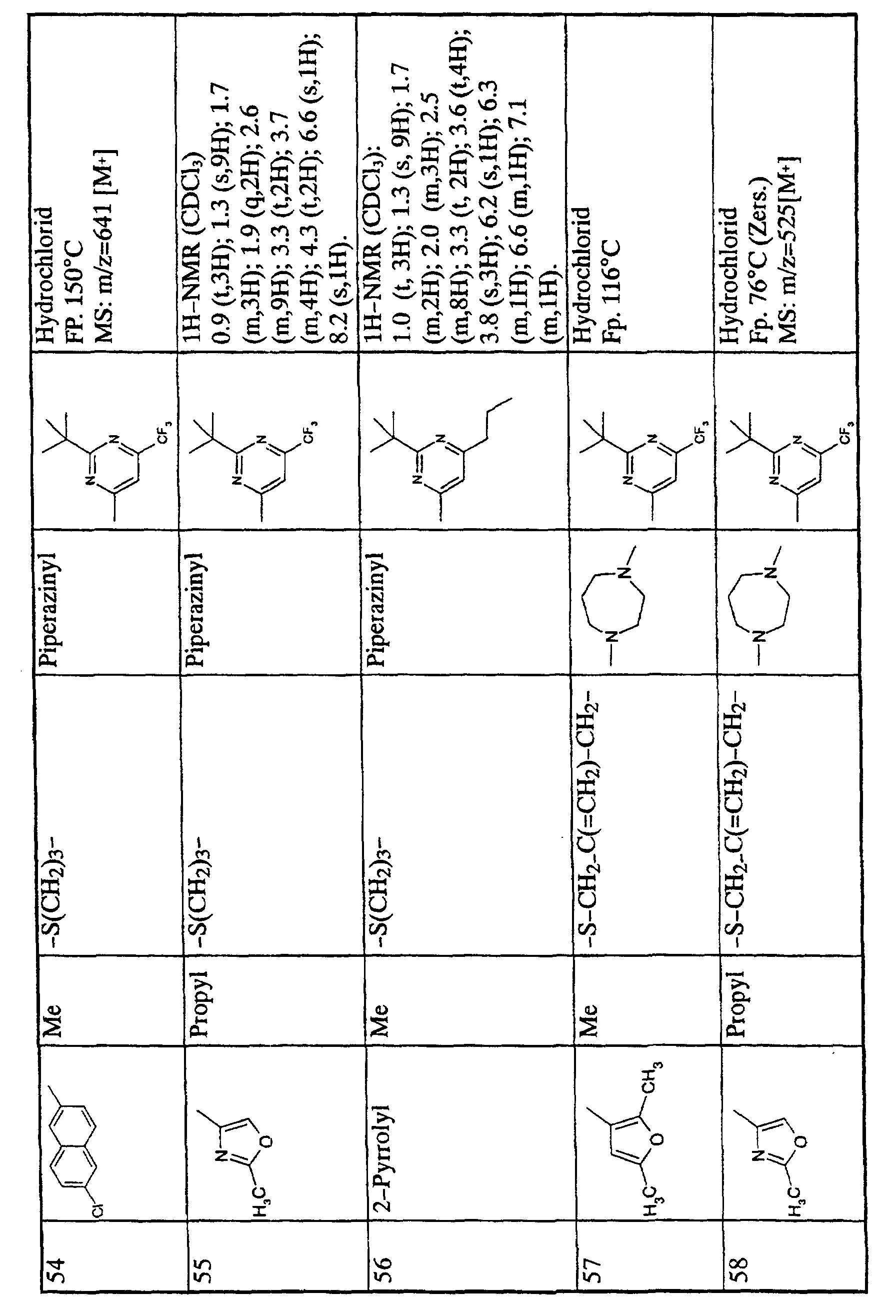

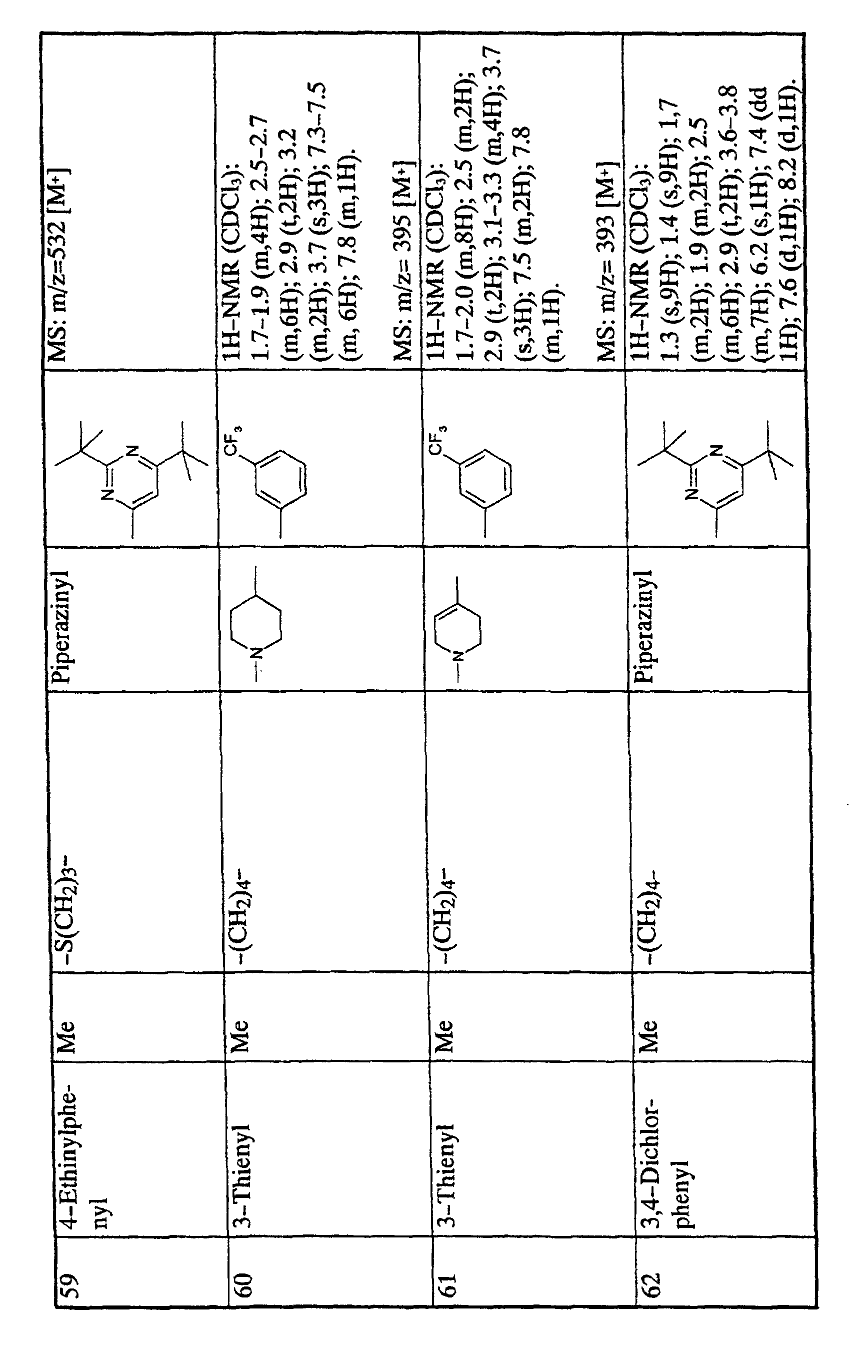

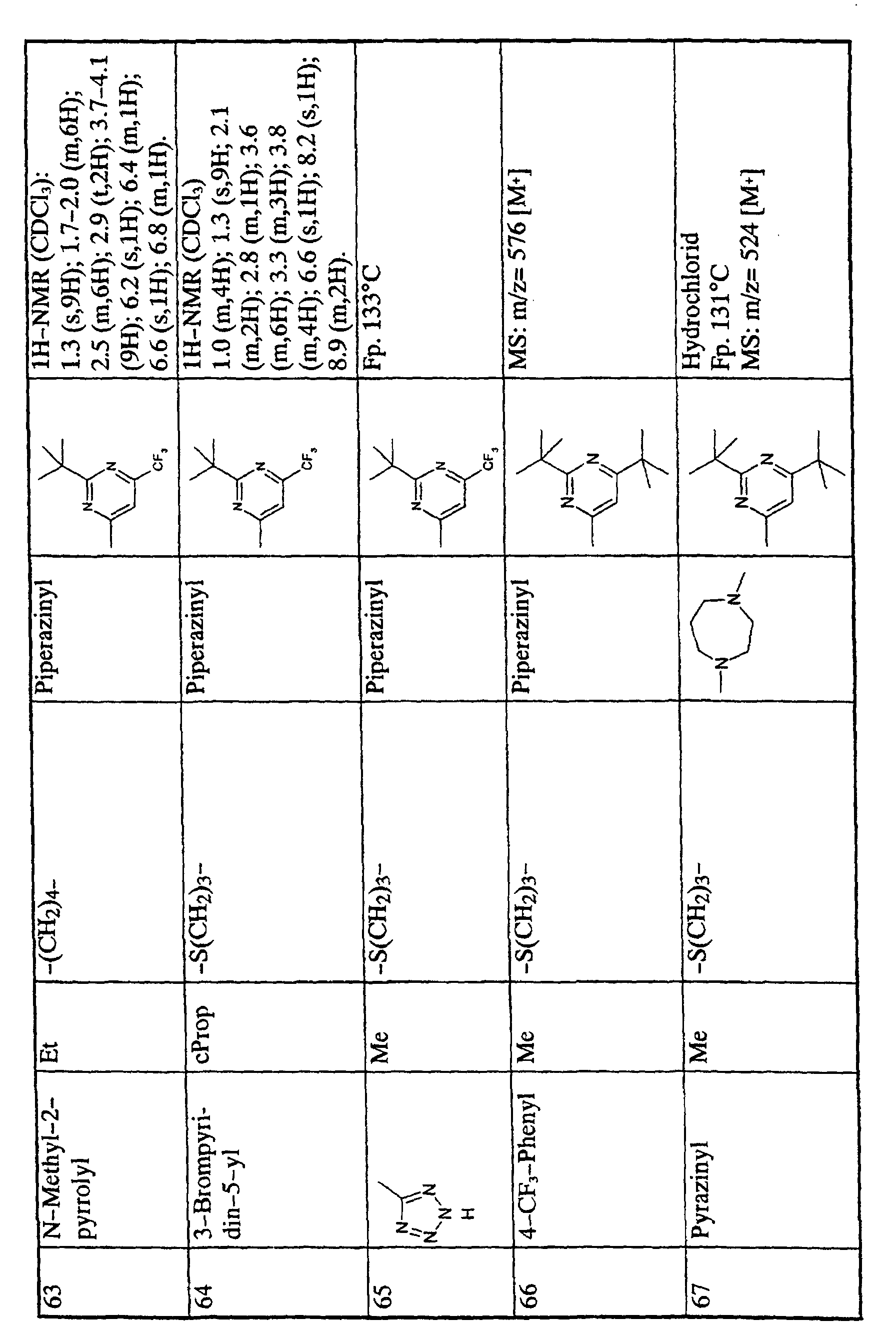

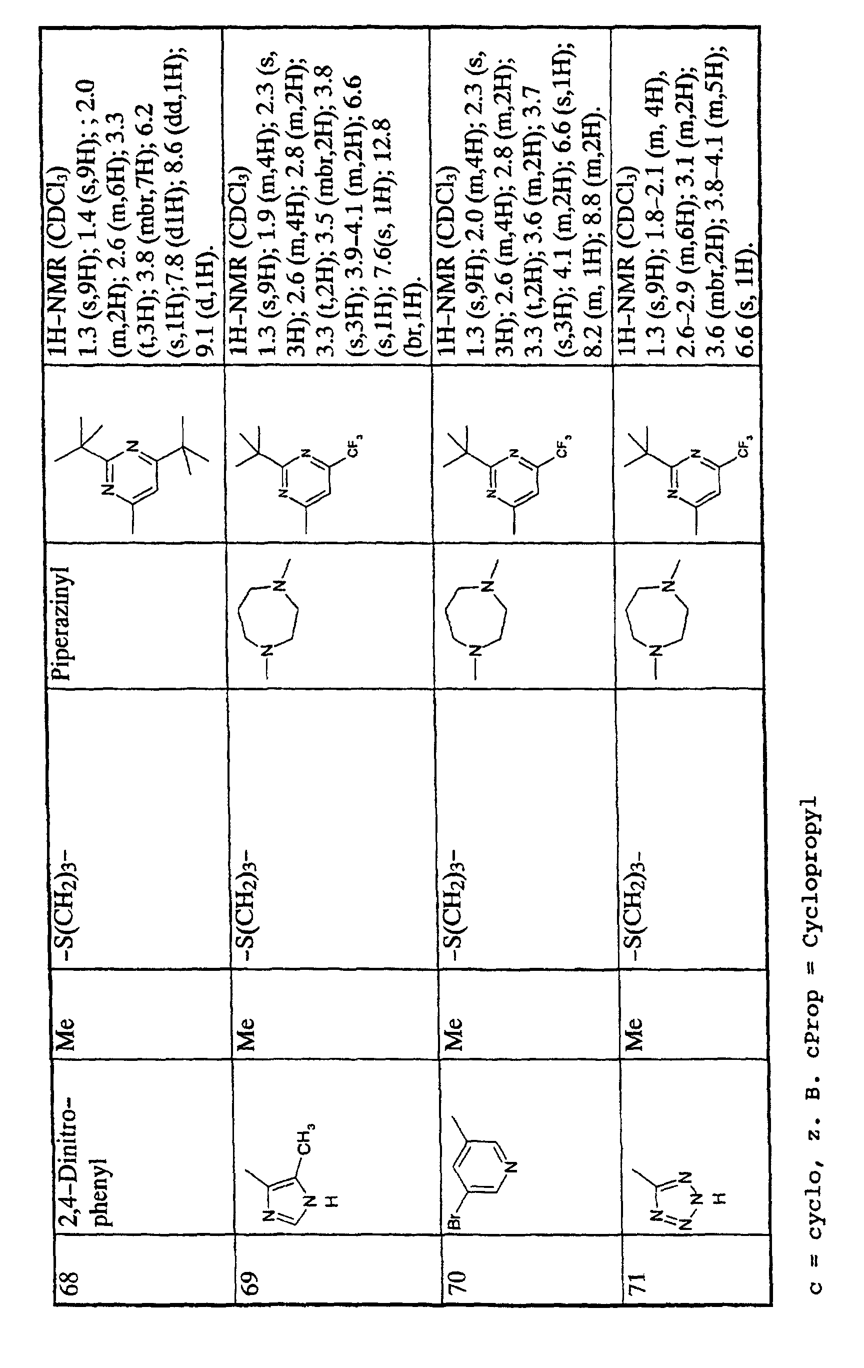

Die Verbindungen der Beispiele 20 - 25 und 65 wurden dabei in folgender Weise erhalten.

### BEISPIEL 20

### 3-{3-[4-(2-t-Butyl-6-trifluormethylpyrimidin-4-yl)-piperazin-1-yl]propoxy}-4-methyl-5-phenyl-1,2,4-(4H)-triazol

855 mg (3mmol) 3-Iod-4-methyl-5-phenyl-1,2,4-(4H)-triazol (hergestellt durch Iodierung von 4-Methyl-5-phenyl-1,2,4-(4H)-triazol analog Izv. Akad. Nauk SSSR, Ser. Khim (1975), 616-619, wurden mit 1,04 g (3 mmol) 2-t-Butyl-4-[4-(3-hydroxypropyl)-piperazin-1-yl]-6-trifluormethylpyrimidin (hergestellt analog Beispiel 1, A.3 durch Umsetzung des nach Beispiel 1, A.2 erhaltenen Produkts mit 3-Chlorpropanol) und Natriumhydrid in DMF 6 h bei 60°C gerührt. Zur Aufarbeitung wurde mit Eiswasser versetzt und mehrfach mit Methyl-t-butylether extrahiert. Der nach Trocknung mit Natriumsulfat und Entfernen des Lösungsmittels erhaltene Rückstand wurde säulenchromatographisch gereinigt (Kieselgel, Methylenchlorid/Methanol). Ausbeute 140 mg (9 % d. Th.) Öl C₂₅H₃₂F₃N₇O (503)
1H-NMR (CDCl₃):
1.3 (s,9H); 2.1 (m,2H); 2.6-2.8 (m,6H); 3.5 (s,3H); 3.8 (mbr,4H); 4.6 (t,2H); 6.5 (s,1H); 7.6 (m,3H); 7.8 (m,2H);

### BEISPIEL 21

### 3-{4-[4-(2,6-Di-t-butylpyrimidin-4-yl)-piperazin-1-yl]-but-1-enyl}-4-methyl-5-phenyl-1,2,4-(4H)-triazol

a. 3-Formyl-4-methyl-5-phenyl-1,2,4-(4H)-triazol
   18,5 g (116 mmol) 4-Methyl-5-phenyl-1,2,4-(4H)-triazol wurden in 235 ml absolutem THF gelöst, auf -70 °C abgekühlt und 85 ml (139 mmol) einer 15%-igen Butyllithiumlösung in Hexan wurden im Verlauf von 15 min bei dieser Temperatur zugetropft. Nach 45 min gab man innerhalb 5 min 72 ml (1,16 mmol) Ameisensäuremethylester zu, wobei die Temperatur auf -50 °C anstieg. Anschließend wurde noch 2 h bei -50 bis -70°C und 30 min bei -25 °C gerührt, sodann mit festem Ammoniumchlorid versetzt, dann Eiswasser zugefügt und 3mal mit Methylenchlorid extrahiert. Nach dem Trocknen und Verdampfen des Lösungsmittels hinterblieben 22,8 g Rückstand, der mittels Flashchromatographie gereinigt wurde (Kieselgel, Essigsäureethylester/Methanol). Ausbeute: 10,9 g (46 % d. Th.)
   C₁₀H₉N₃O (187)
   1H-NMR (CDCl₃) :
   3.9 (s,3H); 7.6 (m,3H); 7.7 (m,2H); 10.2 (s,1H).
b. 3-[4-(2,6-Di-t-butyl-pyrimidin-4-yl)-piperazin-1-yl]-propyltriphenylphosphoniumchlorid
   3,52 g (10 mmol) 1-Chlor-3-[4-(2,6-di-t-butylpyrimidin-4-yl)-piperazin-1-yl]-propan - hergestellt analog Beispiel 1, A.3 - wurden mit 1,8 g Natriumiodid (12 mmol) und 3,41 g (13 mmol) Triphenylphosphin in 75 ml Aceton gelöst und 24 h zum Sieden unter Rückfluß erhitzt.
   Nach dem Abkühlen wurde der Niederschlag abgesaugt, das Filtrat im Vakuum eingedampft und der Rückstand säulenchromatographisch (Kieselgel, Methylenchlorid mit 3,5 % Methanol) gereinigt. Ausbeute: 6,25 g (88 % d. Th.) C₃₇H₄₈IN₄P (706).
   1H-NMR (CDCl₃):
   1.3 (s,9H); 1.4 (s,9H); 1.9 (m,2H); 2.4 (m,4H); 2.7 (m,2H); 3.6 (m,4H); 3.9 (mbr, 2H); 6.3 (s,1H); 7.6-7.9 (m,15H).
c. 5,88 g (8,3 mmol) des vorstehend unter b. hergestellten Phosphoniumsalzes wurden in 15 ml Ethylenglykoldimethylether gelöst, auf 0 °C abgekühlt, und es wurden 280 mg (9,2 mmol) Natriumhydrid zugesetzt und nach 15 min Rühren bei Raumtemperatur, 1,56 g des vorstehend unter a. beschriebenen Aldehyds, gelöst in 10 ml Ethylenglykoldimethylether, bei 0 °C zugetropft.
   Nach 1,5 h Rühren bei Raumtemperatur und weiteren 2 h bei 40 °C wurde mit Toluol und Wasser aufgearbeitet. Vom unlöslichen wurde abfiltriert. Aus der Toluolphase wurden nach Trocknen und Eindampfen 2,6 g Öl erhalten. Ausbeute: roh 65 % d. Th.
   Zur Reinigung wurde das Produkt chromatographiert (Kieselgel, Methylenchlorid/Methanol).
   C₂₉H₄₁N₇ (487).
   1H-NMR (CDCl₃):
   1.3 (s,9H); 1.4 (s,9H); 2.6 (m,8H); 3.7 (m,7H); 6.2 (s,1H); 6.4 (d,1H); 7.0 (td,1H); 7.5 (m,3H); 7.7 (m,2H).

### BEISPIEL 22

### 3-{4-[4-(2,6-Di-t-butylpyrimidin-4-yl)-piperazin-1-yl]-butyl}-4-methyl-5-phenyl-1,2,4-(4H)-triazol

a. 2-[4-Methyl-5-phenyl-1,2,4-(4H)-triazol-3-yl]-1,3-dithian
   6,12 g (32,6 mmol) des nach Beispiel 21 a. hergestellten Aldehyds wurden in 16 ml Chloroform gelöst, sodann wurden bei 0°C 16 ml Essigsäure, 3,28 ml (32,6 mmol) 1,3-Dimercaptopropan und 160 µl Bortrifluorid-Etherat zugegeben. Nach 2,5 h Erhitzen unter Rückfluß wurden nach und nach weitere 2,4 ml Dimercaptopropan und Bortrifluorid-Etherat zugesetzt und weitere 6 h erhitzt, bis vollständiger Umsatz des Aldehyds erreicht war.
   Nach dem Abkühlen auf 0 °C wurde mit 10%-iger Natronlauge auf pH 9-10 eingestellt, 1 h bei 0 °C gerührt, sodann wurde 3mal mit Methylenchlorid extrahiert. Aus der getrockneten und eingedampften Lösungsmittelphase wurden 13,2 g eines gelben Öls erhalten, die säulenchromatographisch gereinigt wurden (Kieselgel, Essigsäureethylester). Ausbeute: 4,3 g (48 % d. Th.), farbloser Feststoff.
   C₁₃H₁₅N₃S₂ (277).
   1H-NMR (CDCl₃):
   2.1 (m,2H); 2.9 (m,2H); 3.3 (m,2H); 3.7 (s,3H); 5.3 (s,1H); 7.5 (m,3H); 7.7 (m,2H).
b. 831 mg (3 mmol) des vorstehend beschriebenen Dithians wurden in 7,5 ml trockenem THF gelöst und bei -70 °C mit 2,2 ml (3,6 mmol) einer 15%-igen Lösung von Butyllithium in n-Hexan behandelt. Nach 60 min Rühren bei -70 °C bis -50 °C wurden 1,06 g (3 mmol) 1-Chlor-3-[4-(2,6-di-t-butylpyrimidin-4-yl)-piperazin-1-yl]-propan- hergestellt analog Beispiel 1, A.3 - gelöst in 5 ml THF zugetropft. Man erwärmte nun langsam auf Raumtemperatur und erwärmte noch 60 min auf 30 bis 50 °C, um vollständigen Umsatz zu erzielen. Zur Aufarbeitung wurde dem erkalteten Ansatz festes Ammoniumchlorid zugefügt. Der Ansatz wurde sodann auf Eis/Wasser gegeben und mehrfach mit Methylenchlorid und Methyl-t-butylether extrahiert. Nach Trocknen und Einengen hinterblieben 1,74 g (98 % d. Th.) des substituierten Dithians, das anschließend mit Raney-Nickel und Wasserstoff bei 40 °C im Verlauf von 12 h in Tetrahydrofuran hydriert wurde. Nach dem Abtrennen des Katalysators wurde der Rückstand chromatographisch gereinigt (Kieselgel, Methylenchlorid/Methanol). Ausbeute: 700 mg (49 % d. Th.).
   Farblose Festsubstanz, Fp. 144-145 °C.
   C₂₉H₄₃N₇ (489).

### BEISPIEL 23

### 3-{4-[4-(2-t-Butyl-6-trifluormethylpyrimidin-4-yl)-piperazin-1-yl]-butyl}-4-methyl-5-phenyl-1,2,4-(4H)-triazol-Hydrochlorid

Die Verbindung wurde analog Beispiel 22 unter Verwendung der Chlorverbindung aus Beispiel 1, A.3 dargestellt.
C₂₉H₃₄F₃N₇ (502)
1H-NMR (CDCl₃):
1.3 (s,9H); 1.7 (m,2H); 1.9 (q,2H); 2.4 (t,2H); 2.5 (t,4H); 2.8 (t,2H); 3.6 (s,3H); 3.75 (m,4H); 6.6 (s,1H); 7.4 (m,3H); 7.6 (m,2H).

### BEISPIEL 24

### 3-{3-[4-(2-t-Butyl-6-trifluormethylpyrimidin-4-yl)-piperazin-1-yl]-propylmercapto}-5-(2,5-dimethylfuran-3-yl)-4-methyltriazol-Hydrochlorid

Durch Umsetzung von 2,5-Dimethylfuran-3-carbonsäurechlorid mit N-Methylthiosemicarbazid und anschließender Cyclisierung nach der Methode von Kubota und Uda, Chem. Pharm. Bull. (1975), 23, 955-966, wurde 2,5-Dimethylfuran-3-yl-3-mercapto-4-methyl-1,2,4-(4H)-triazol erhalten.
C₉H₁₁N₃OS (209).
1H-NMR (CDCl₃):
2.2 (s,3H); 2.3 (s,3H); 3.5 (s,3H); 6.5 (1H).

Durch Umsetzung analog Beispiel 1B wurde die oben genannte Verbindung erhalten. Fp. 190-192 °C
C₂₅H₃₄F₃N₇OS HCl (574)

### BEISPIEL 25

### 3-{3-[4-2-t-Butyl-6-trifluormethylpyrimidin-4-yl)-piperazin-1-yl]-propylmercapto}-5-(pyrazin-2-yl)-4-methyltriazol-Hydrochlorid

Durch Umsetzung von Pyrazin-2-carbonsäurechlorid analog der Methode von Kubota und Uda in Beispiel 24 wurde 3-Mercapto-4-methyl-5-pyrazin-2-yl-1,2,4-(4H)-triazol erhalten.

Die oben genannte Verbindung wurde ebenfalls analog Beispiel 1B hergestellt. Fp. 164-169 °C.
C₂₃H₃₁F₃N₉ (522).

### BEISPIEL 65

### 3-(3-(4-(2-t-Butyl-6-trifluormethylpyrimidin-4-yl)piperazin-1-yl)propylmercapto-4-methyl-5-(1H)-tetrazolyl-5)-1,2,4(4H)-triazol.

a) 3-(3-(4-(2-t-Butyl-6-trifluormethylpyrimidin-4-yl)piperazin-1-yl)propylmercapto-4-methyl-1,2,4(4H)-triazol-5-carbonsäureamid
   950 mg (6,0 mmol) 5-Mercapto-4-methyl-1,2,4(4H)-triazol-3-carbonsäureamid wurden mit 2,2 g (6,0 mmol) der nach Beispiel 1.A3 hergestellten Chlorbase und 144 mg Lithiumhydroxid (6,0 mmol) in 17 ml DMF 3 h unter Rühren auf 100 °C erwärmt. Nach dem Abkühlen wurde mit 100 ml Wasser versetzt und mit Methyl-t-butylether extrahiert, die Lösungsmittelphase getrocknet und eingedampft. Der Rückstand wurde chromatographisch gereinigt (Kieselgel, Methylenchlorid-Methanol 95:5).
   Ausbeute: 1,65 g (57 % d.Th.)
   FP 141-143 °C
   C₂₀H₂₉F₃N₈OS (MG 486)
b) 3-(3-(4-(2-t-Butyl-6-trifluormethyl-pyrrimidin-4-yl)piperazin-1-yl)propylmercapto-5-cyano-4-methyl-1,2,4(4H)-triazol.
   1,15 g (24,0 mmol) der vorstehend beschriebenen Verbindung wurden in 20 ml Methylenchlorid und 2 ml (12,0 mmol) Dipropylethylamin gelöst, auf 0 °C abgekühlt und langsam mit 0,5 ml Trifluoracetanhydrid versetzt. Nach 3 h Rühren bei Raumtemperatur wurde zweimal mit Wasser, dann mit 20-prozentiger NaHSO₄-Lösung, gesättigter NaHCO₃-Lösung und Kochsalz-Lösung gewaschen, die organische Phase getrocknet und eingedampft. Es hinterblieben 0,9 g Öl (81 % d.Th.). Eine Probe wurde mit etherischer Salzsäure in das Hydrochlorid überführt.
   Fp 220 - 222 °C
   C₂₀H₂₇F₃N₈S (MG 468)
   C₂₀H₂₈ClF₃N₈S (MG 503,5)
c) 5-(3-(4-(2-t-Butyl-6-trifluormethyl-pyrimidin-4-yl)piperazin-1-yl)propylmercapto-4-methyl-3-((1H)-tetrazolyl-5)-1,2,4(4H)-triazol.
   Man löste 0,8 g (1,7 mmol) der vorstehend beschriebenen Substanz in 1 ml DMF, gab 122 mg (1,9 mmol) Natriumazid und 100 mg (1,9 mmol) Ammoniumchlorid zu und erwärmte 2 h unter Rühren auf 85 °C. Zur Aufarbeitung wurde mit wenig Wasser versetzt, die Lösung mit NaOH auf pH 7 eingestellt und mit Methylenchlorid extrahiert. Nach Trocknen und Einengen wurde ca. 1 g Rückstand erhalten, der säulenchromatographisch gereinigt wurde. (Kieselgel, Methylenchlorid-Methanol 8:2).
   Ausbeute: 0,38 g (43% d. Th)
   Fp. 133° (Zersetzung)
   C₂₀H₂₈F₃N₁₁S (MG 511)

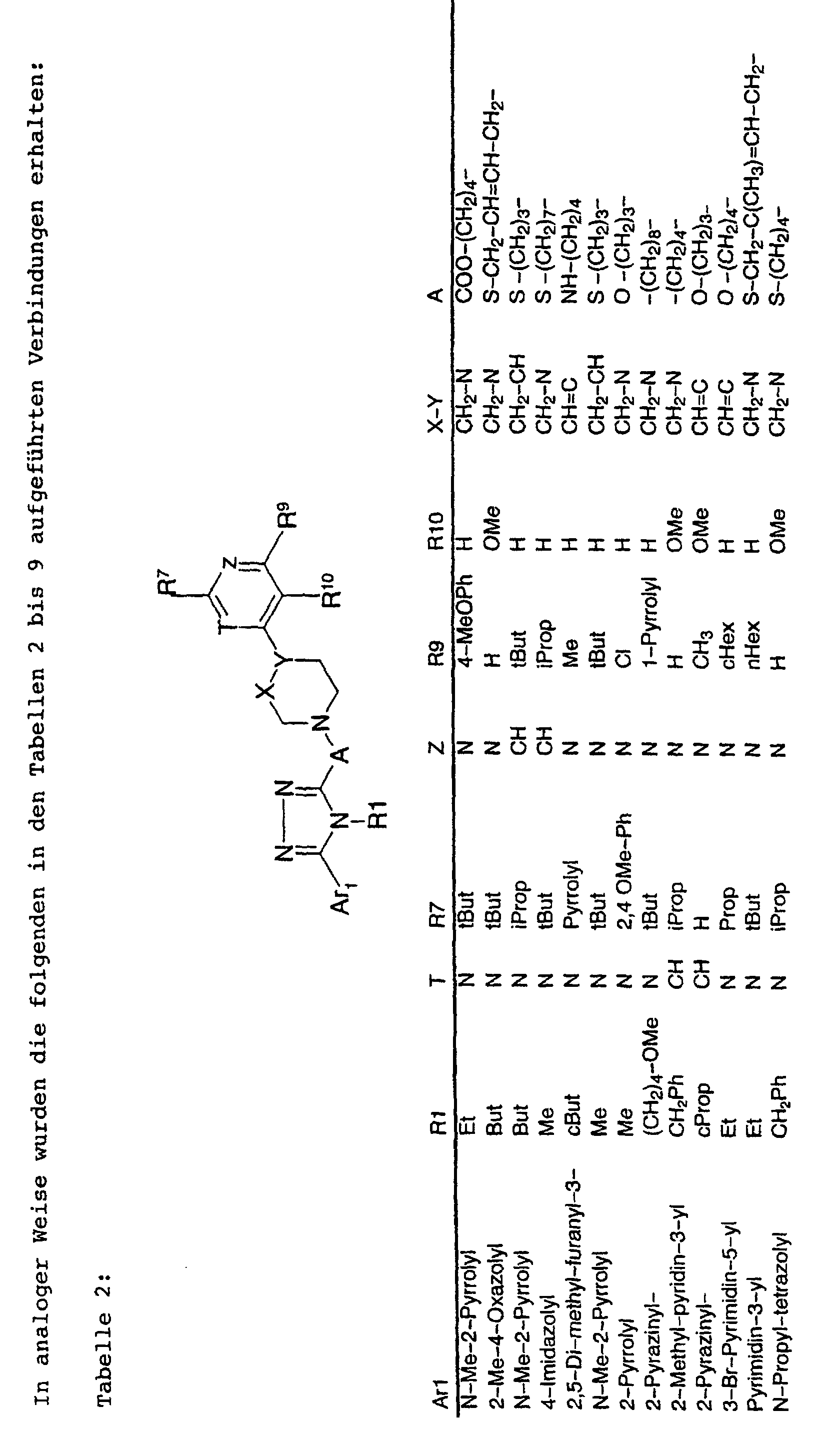

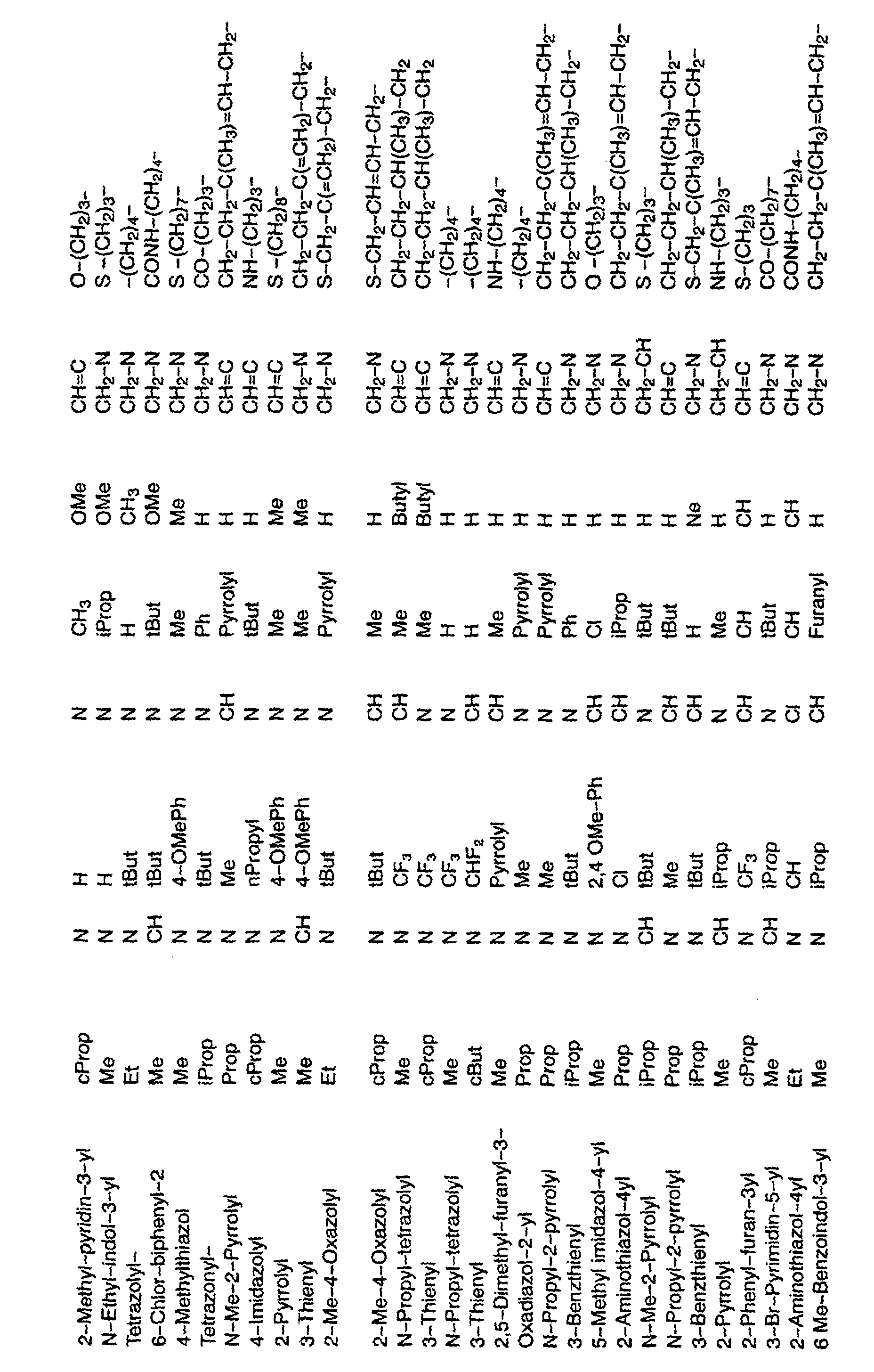

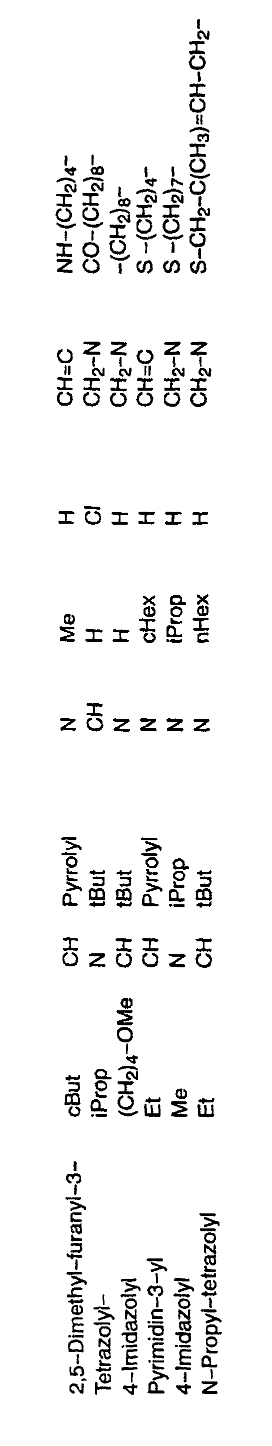

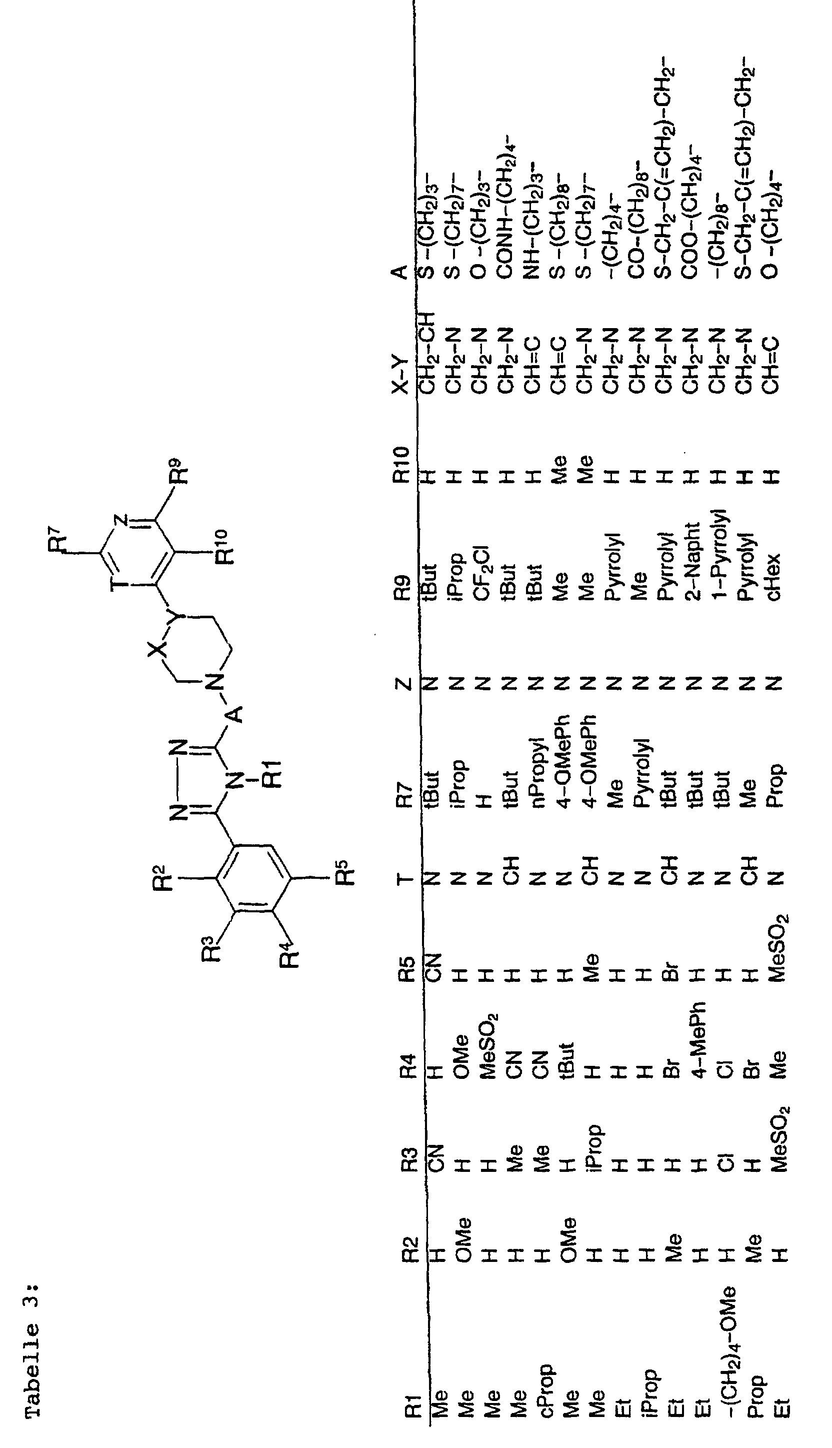

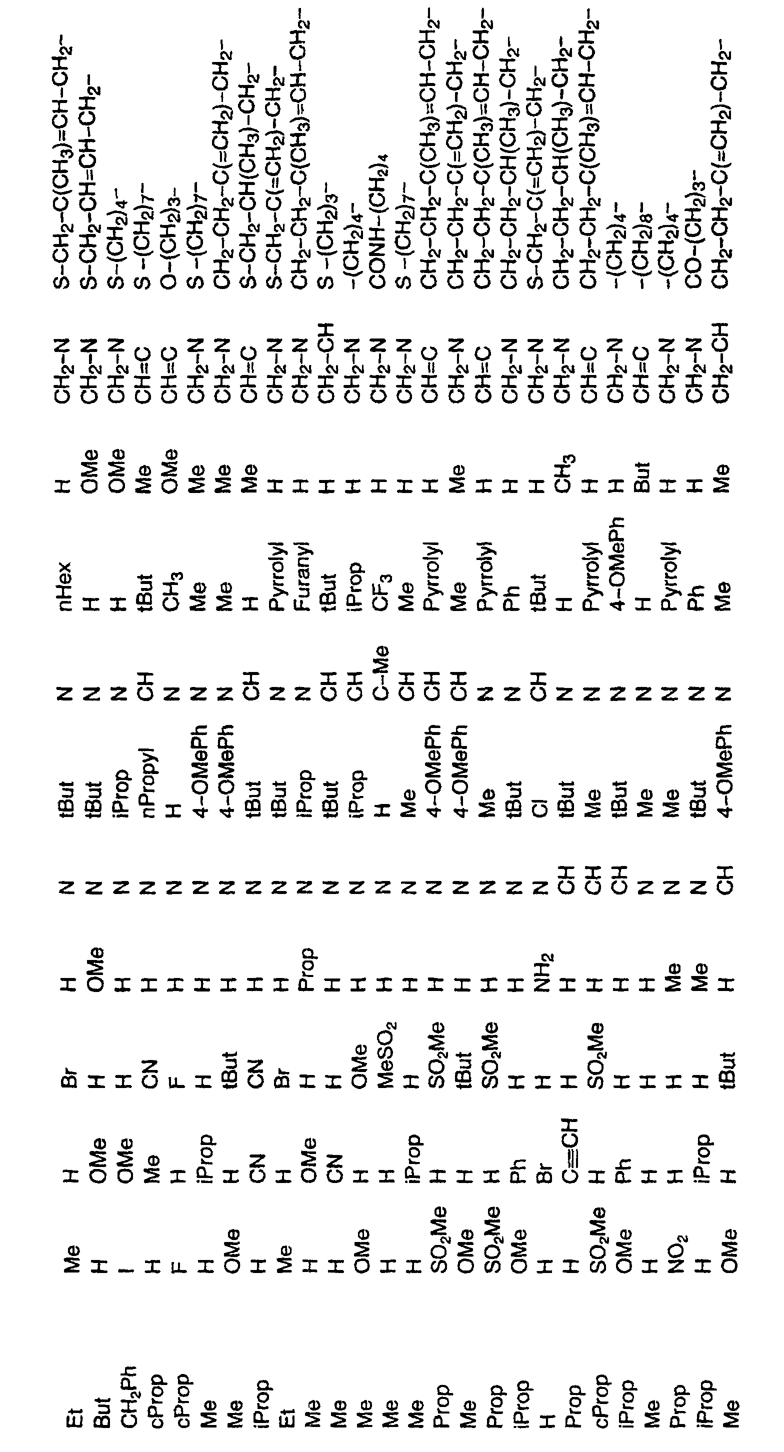

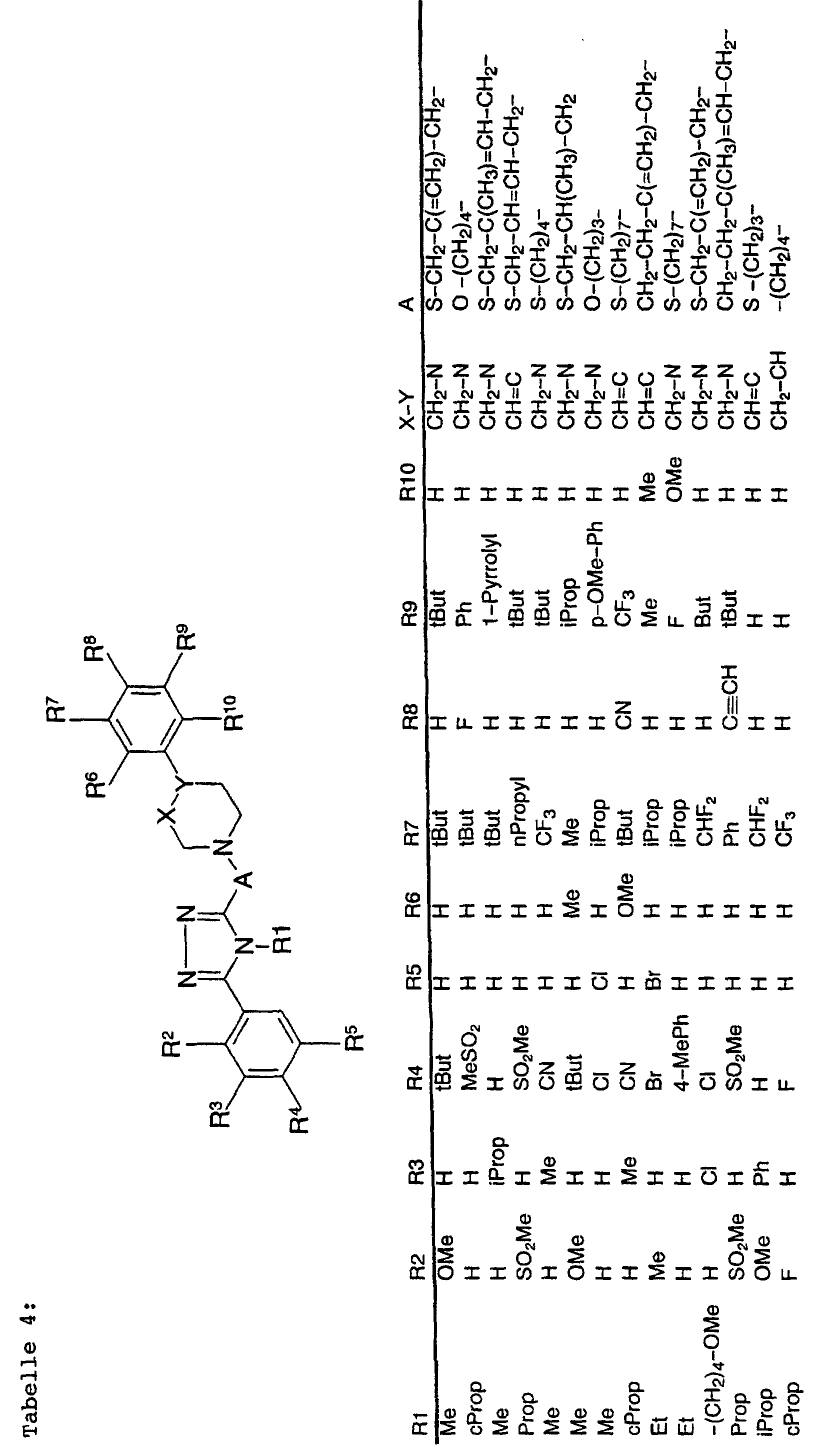

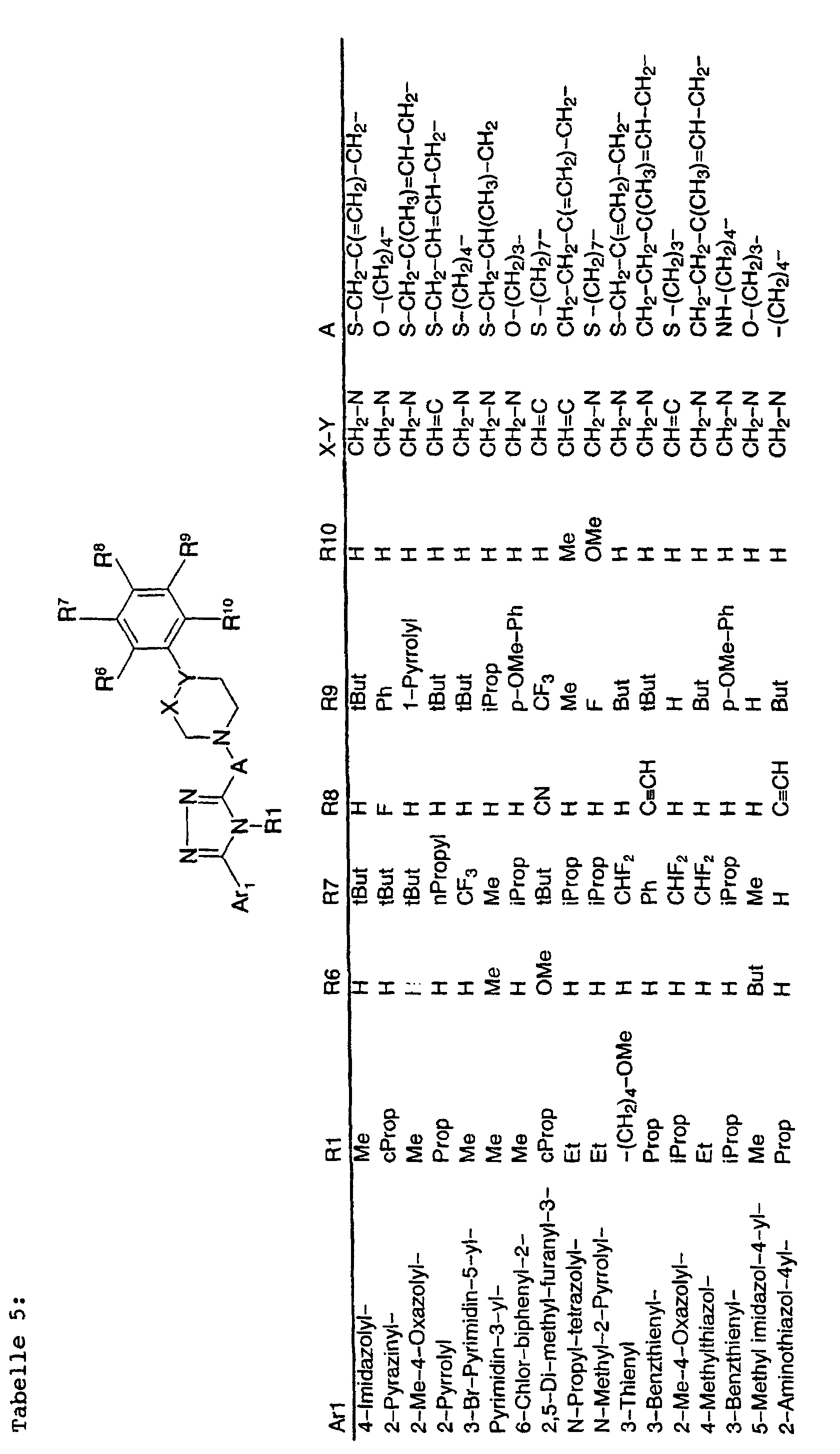

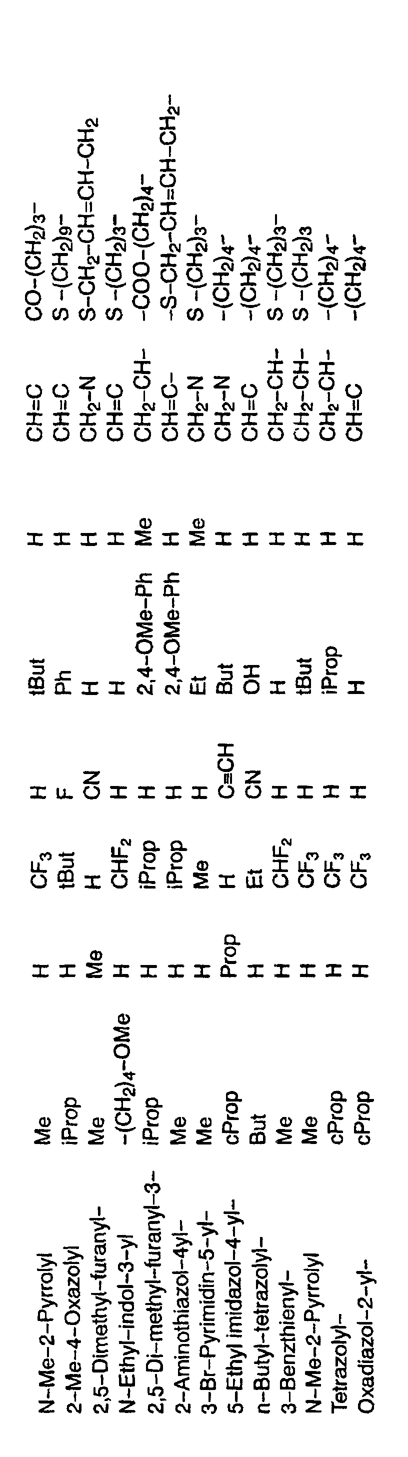

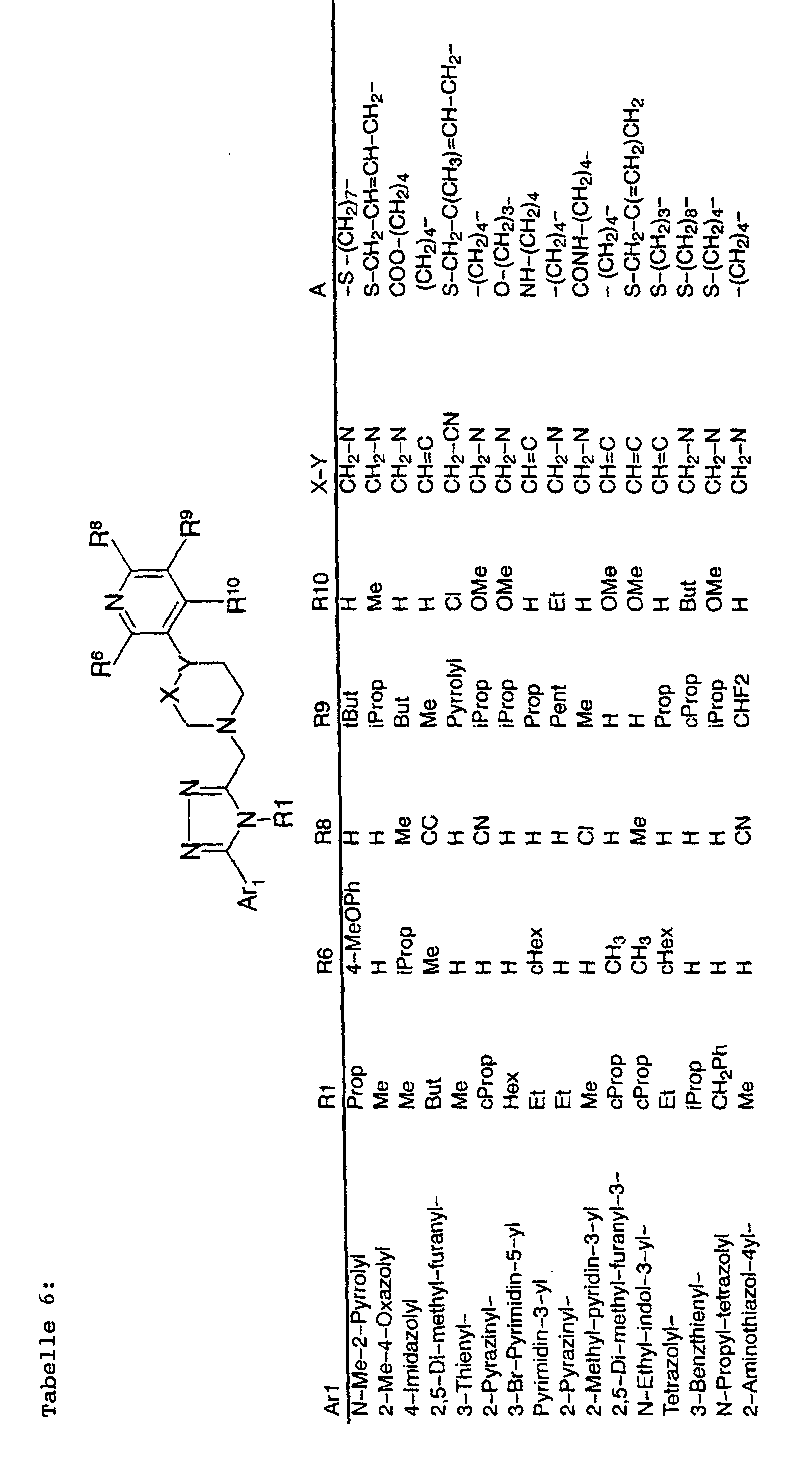

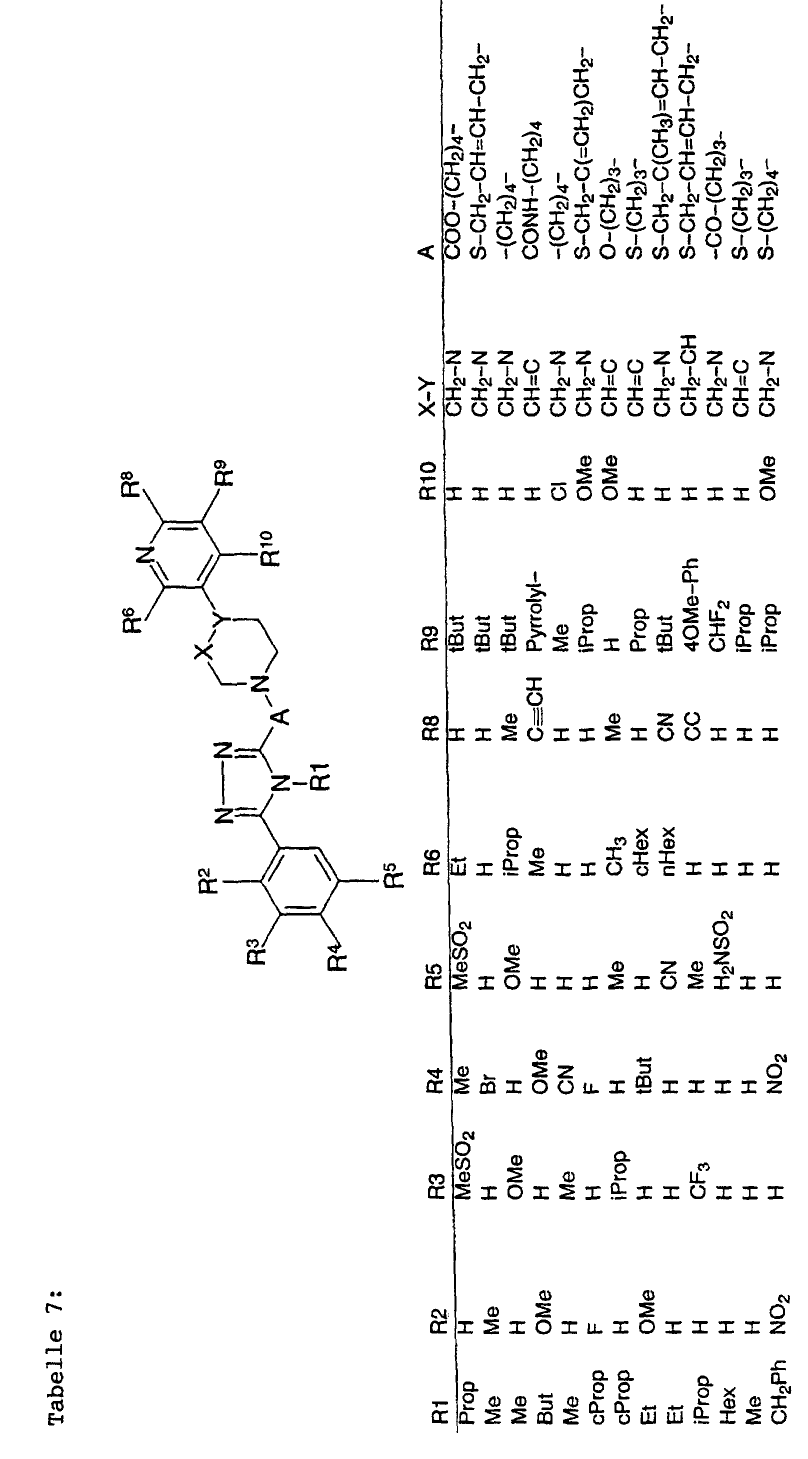

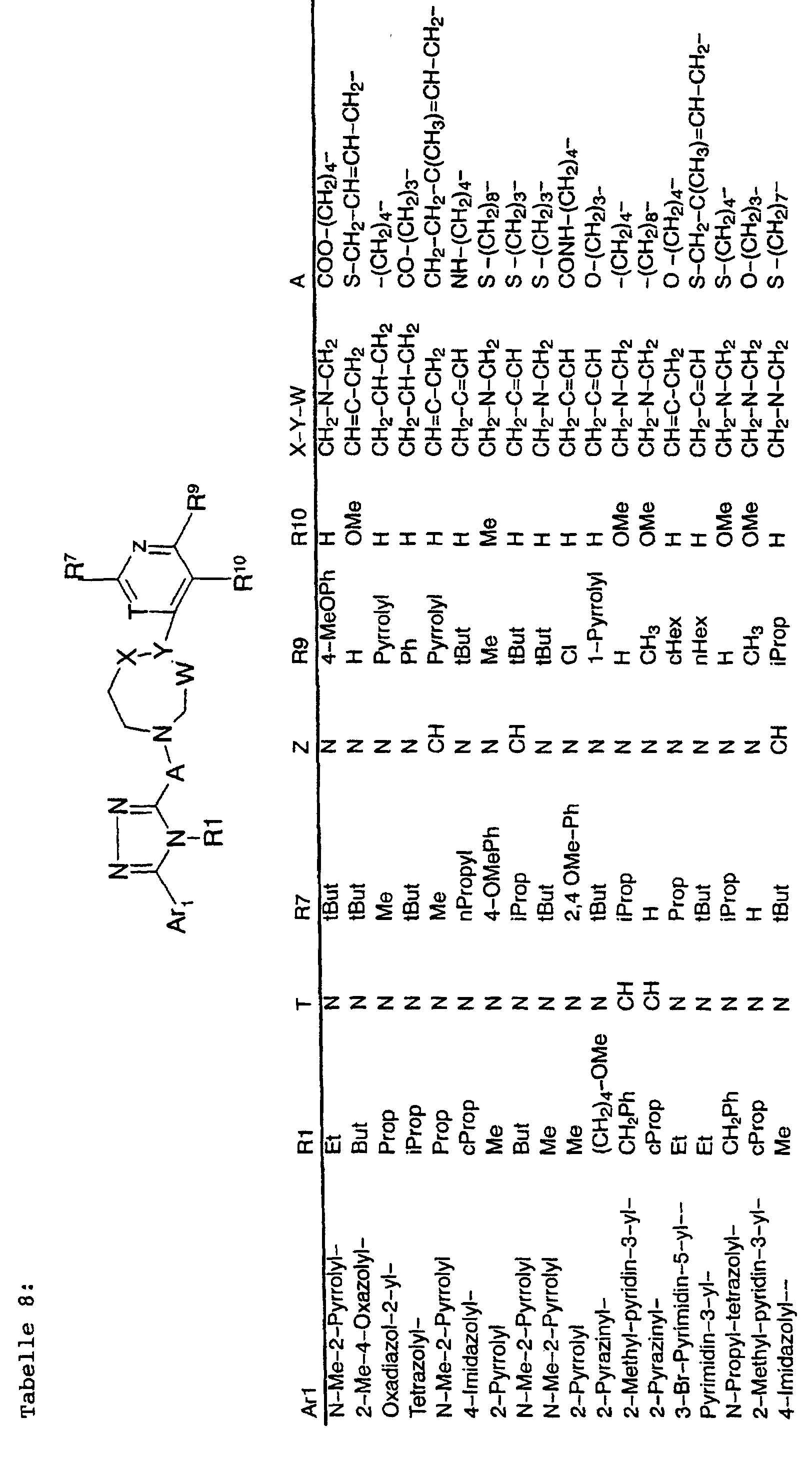

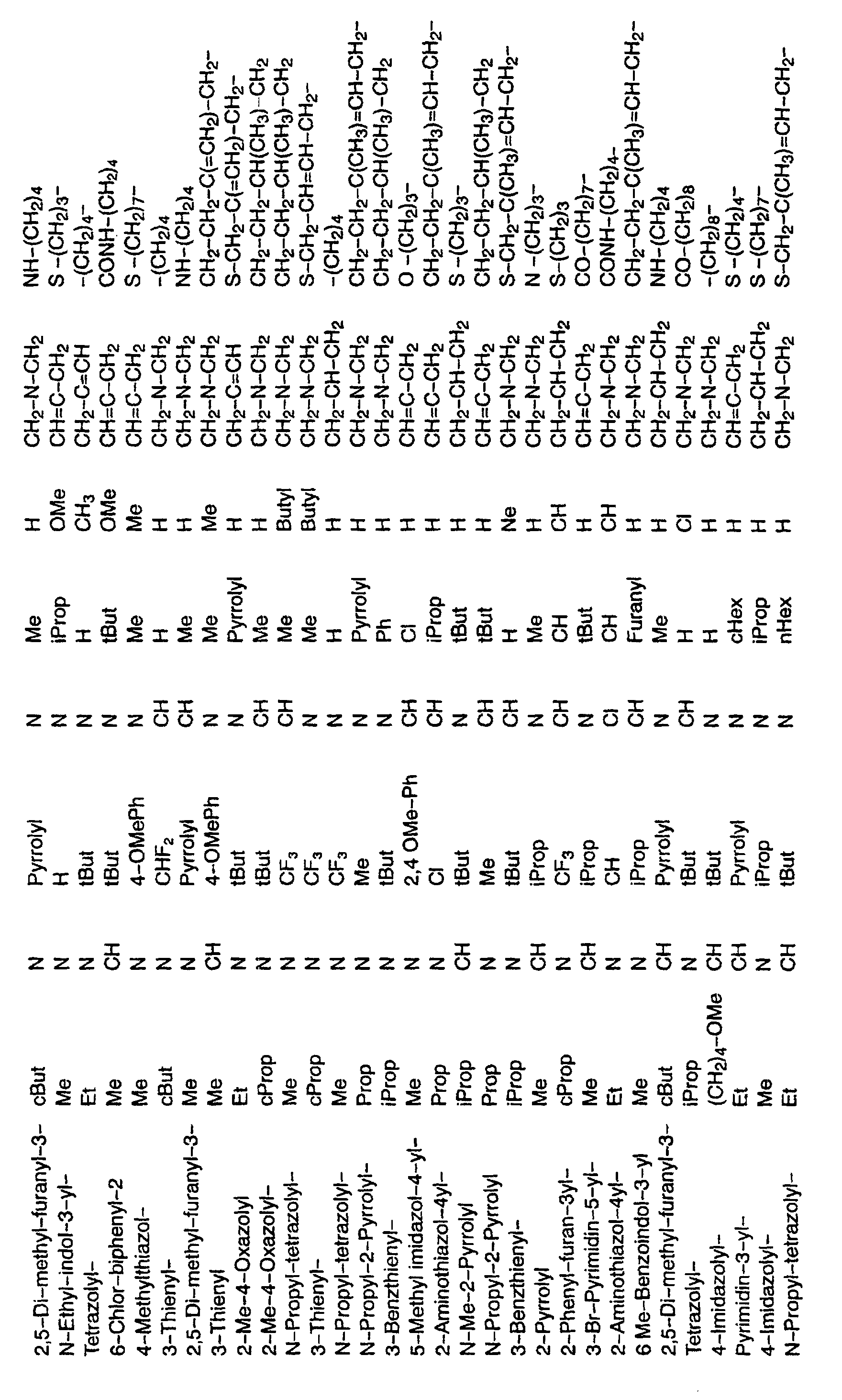

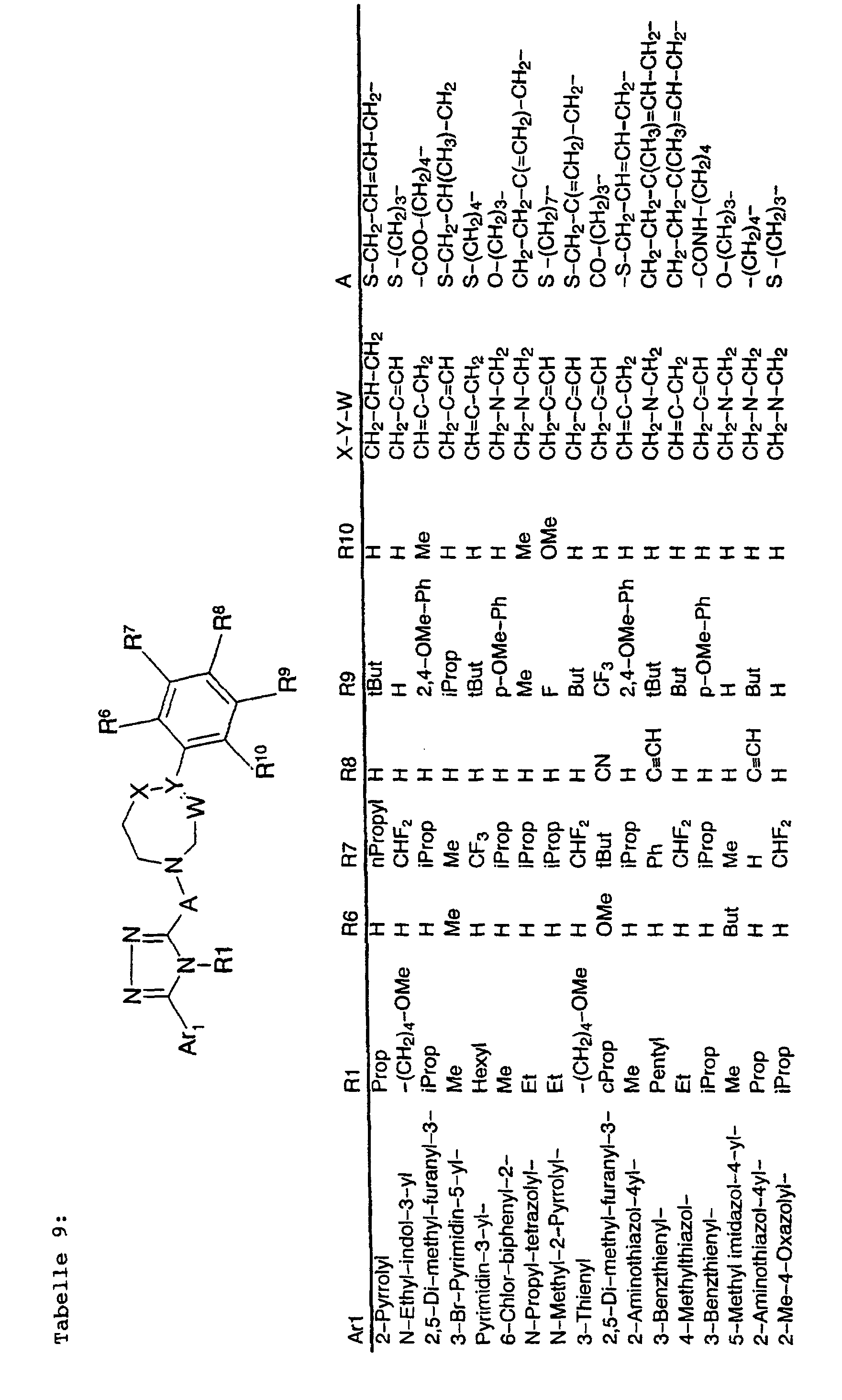

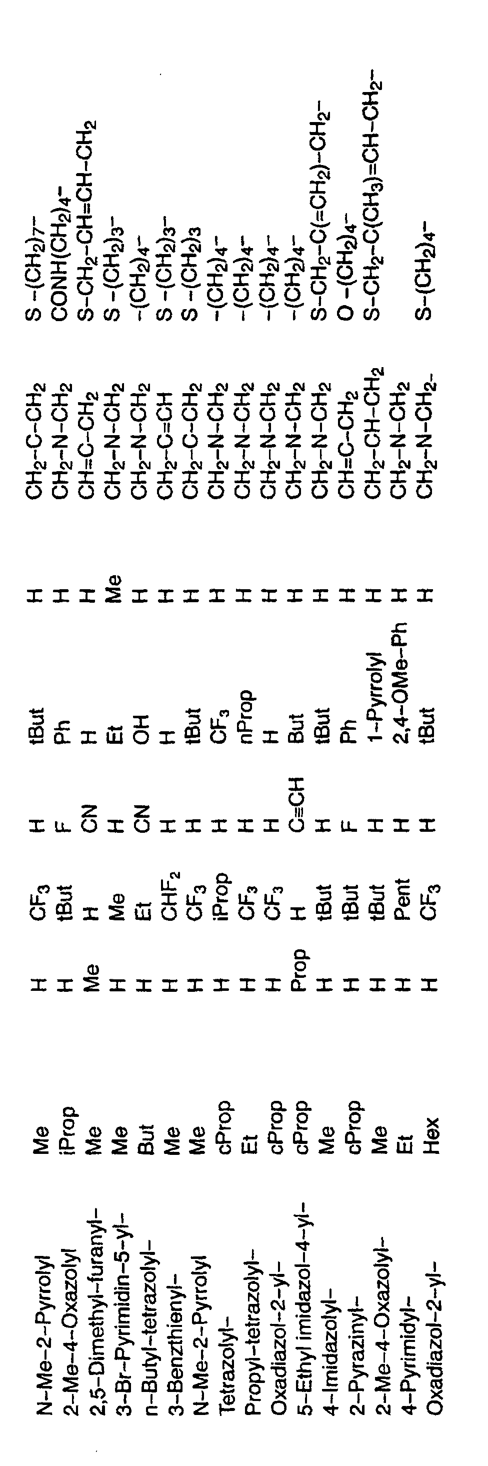

### Beispiele für galenische Applikationsformen

### A) Tabletten

Auf einer Tablettenpresse werden in üblicher Weise Tabletten folgender Zusammensetzung gepreßt:
- 40 mg: Substanz des Beispiels 1
- 120 mg: Maisstärke
- 13,5 mg: Gelatine
- 45 mg: Milchzucker
- 2,25mg: Aerosil® (chemisch reine Kieselsäure in submikroskopisch feiner Verteilung)
- 6,75mg: Kartoffelstärke (als 6 %iger Kleister)

### B) Dragees

- 20 mg: Substanz des Beispiels 4
- 60 mg: Kernmasse
- 70 mg: Verzuckerungsmasse

Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus

5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### Biologische Untersuchungen - Rezeptorbindungsstudien

### 1) D₃-Bindungstest

Für die Bindungsstudien wurden klonierte humane D₃-Rezeptorexprimierende CCL 1,3 Mäusefibroblasten, erhältlich bei Res. Biochemicals Internat. One Strathmore Rd., Natick, MA 01760-2418 USA, eingesetzt.

### Zellpräparation

Die D₃ exprimierenden Zellen wurden in RPMI-1640 mit 10 % fötalem Kälberserum (GIBCO Nr. 041-32400 N); 100 E/ml Penicillin und 0,2 % Streptomycin (GIBCO BRL, Gaithersburg, MD, USA) vermehrt. Nach 48 h wurden die Zellen mit PBS gewaschen und mit 0,05 % trypsinhaltiger PBS 5 min inkubiert. Danach wurde mit Medium neutralisiert und die Zellen durch Zentrifugation bei 300 g gesammelt. Zur Lyse der Zellen wurde kurz das Pellet mit Lysispuffer (5mM Tris-HCl, pH 7,4 mit 10 % Glycerin) gewaschen und danach in einer Konzentration von 10⁷-Zellen / ml Lysispuffer 30 min bei 4 °C inkubiert. Die Zellen wurden bei 200 g 10 min zentrifugiert und das Pellet in flüssigem Stickstoff gelagert.

### Bindungstests

Für den D₃-Rezeptorbindungstest wurden die Membranen in Inkubationspuffer (50 mM Tris-HCl, pH 7,4 mit 120 mM NaCl, 5 mM KCl, 2 mM CaCl₂, 2 mM MgCl₂, 10µM Quinolinol, 0,1 % Ascorbinsäure und 0,1 % BSA) in einer Konzentration von ca. 10⁶ Zellen/250 µl Testansatz suspendiert und bei 30 °C mit 0,1 nM ¹²⁵Jodsulpirid in Anwesenheit und Abwesenheit von Testsubstanz inkubiert. Die unspezifische Bindung wurde mit 10⁻⁶M Spiperon bestimmt.

Nach 60 min wurde der freie und der gebundene Radioligand durch Filtration über GF/B Glasfaserfilter (Whatman, England) an einem Skatron-Zellsammler (Skatron, Lier, Norwegen) getrennt und die Filter mit eiskaltem Tris-HCl-Puffer, pH 7,4 gewaschen. Die auf den Filtern gesammelte Radioaktivität wurde mit einem Packard 2200 CA Flüssigkeitszintillationszähler quantifiziert.

Die Bestimmung der Kᵢ-Werte erfolgte über nichtlineare Regressionsanalyse mit dem Programm LIGAND.

### 2) D₂-Bindungstest

### Zellkultur

HEK-293 Zellen mit stabil exprimierten humanen Dopamin-D2A-Rezeptoren wurden in RPMI 1640 mit Glutamax I™ und 25 mM HE-PES mit 10% fötalem Kälberserumalbumin kultiviert. Alle Medien enthielten 100 Einheiten pro ml Penicillin und 100 µg/ml Streptomycin. Die Zellen wurden in feuchter Atmosphäre mit 5% CO₂ bei 37 °C gehalten.

Die Zellpräparation für Bindungsstudien erfolgte durch Trypsinisierung (0,05% Trypsinlösung) für 3-5 Minuten bei Raumtemperatur. Danach wurden die Zellen bei 250 g 10 Minuten zentrifugiert und 30 Minuten bei 4 °C mit Lysispuffer (5 mM Tris-HCl, 10% Glycerol, pH 7,4) behandelt. Nach Zentrifugation bei 250 g für 10 Minuten wurde der Rückstand bei -20 °C bis zum Gebrauch aufbewahrt.

### Rezeptorbindungstests

Dopamin-D₂-Rezeptor "low affinity state" mit ¹²⁵I-Spiperon (81 TBq/mmol, Du Pont de Nemours, Dreieich)

Die Ansätze (1 ml) setzten sich zusammen aus 1 x 10⁵ Zellen in Inkubationspuffer (50 mM Tris, 120 mM NaCl, 5 mM KCl, 2 mM MgCl₂ und 2 mM CaCl₂, pH 7,4 mit HCl) und 0,1 nM ¹²⁵I-Spiperon (totale Bindung) oder zusätzlich 1 µM Haloperidol (unspezifische Bindung) oder Prüfsubstanz.

Nach erfolgter Inkubation bei 25 °C für 60 Minuten wurden die Ansätze über GF/B Glasfaserfilter (Whatman, England) an einem Skatron-Zellsammler (Fa. Zinsser, Frankfurt) filtriert und die Filter mit eiskaltem 50 mM Tris-HCl-Puffer, pH 7,4 gewaschen. Die auf den Filtern gesammelte Radioaktivität wurde mit einem Packard 2200 CA Flüssigkeitszintillationszähler quantifiziert.

Die Auswertung erfolgte wie unter a).

Die Bestimmung der Kᵢ-Werte erfolgte über nichtlineare Regressionsanalyse mit dem Programm LIGAND oder durch Umrechnung der IC₅₀-Werte mit Hilfe der Formel von Cheng und Prusoff.

Die erfindungsgemäßen Verbindungen zeigen in diesen Tests sehr gute Affinitäten am D₃-Rezeptor (< 1 µmolar, insbesondere < 100 nmolar) und hohe Selektivitäten gegenüber dem D₃-Rezeptor.

## Patentansprüche

1. Triazolverbindungen der Formel I 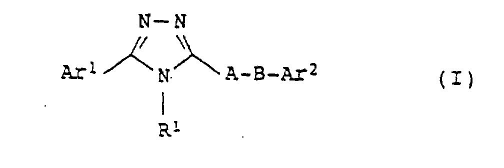 worin
Ar¹ für Phenyl, Naphthyl oder einen 5- oder 6-gliedrigen heterocyclischen aromatischen Ring mit 1 bis 4 Heteroatomen, die unabhängig voneinander ausgewählt sind unter O, S und N, steht, wobei Ar¹ gegebenenfalls 1, 2, 3 oder 4 Substituenten aufweist, die unabhängig voneinander ausgewählt sind unter C₁-C₆-Alkyl, das gegebenenfalls durch OH, OC₁-C₆-Alkyl, Halogen oder Phenyl substituiert ist, C₁-C₆-Alkoxy, C₂-C₆-Alkenyl, C₂-C₆-Alkinyl, C₃-C₆-Cycloalkyl, Halogen, CN, COOR², NR²R², NO₂, SO₂R², SO₂NR²R² und Phenyl, das gegebenenfalls durch C₁-C₆-Alkyl, OC₁-C₆-Alkyl, NR²R², CN, CF₃, CHF₂, oder Halogen substituiert ist und wobei der erwähnte heterocyclische, aromatische Ring gegebenenfalls mit einem Phenylring auch kondensiert sein kann;
A für geradkettiges oder verzweigtes C₄-C₁₀-Alkylen oder geradkettiges oder verzweigtes C₃-C₁₀-Alkylen steht, das wenigstens eine Gruppe Z umfasst, die ausgewählt ist unter O, S, NR², CONR², COO, CO, einer Doppel- oder Dreifachbindung,
B für einen Rest der Formel steht: 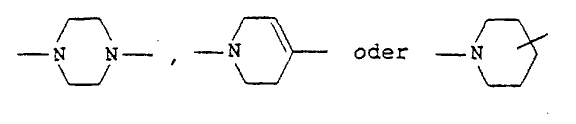 oder wenn Ar¹ für den 5- oder 6-gliedrigen, heterocyclischen aromatischen Ring, der wie angegeben substituiert sein kann, steht, B auch für einen Rest der Formeln 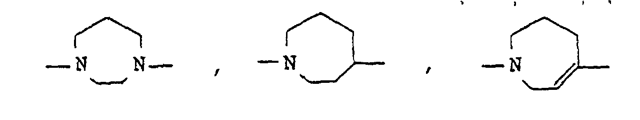 oder 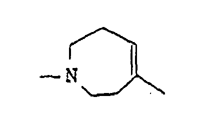 stehen kann,
Ar² für Phenyl, Pyridyl, Pyrimidinyl oder Triazinyl steht, wobei Ar² gegebenenfalls ein bis vier Substituenten aufweisen kann, die unabhängig voneinander ausgewählt sind unter OR², C₁-C₆-Alkyl, C₂-C₆-Alkenyl, C₂-C₆-Alkinyl, C₁-C₆-Alkoxy-C₁-C₆-alkyl, Halogen-C₁-C₆-alkyl, Halogen-C₁-C₆-alkoxy, Halogen, CN, NO₂, SO₂R², NR²R², SO₂NR²R², einem 5- oder 6-gliedrigen carbocyclischen, aromatischen oder nicht-aromatischen Ring und einem 5- oder 6-gliedrigen, heterocyclischen aromatischen oder nicht-aromatischen Ring mit 1 oder 2 Heteroatomen, die ausgewählt sind unter O, S und N, wobei der carbocyclische oder heterocyclische Ring gegebenenfalls durch C₁-C₆-Alkyl, Phenyl, Phenoxy, Halogen, OC₁-C₆-Alkyl, OH, NO₂ oder CF₃ substituiert und/ oder mit einem Phenylring kondensiert sein kann und wobei Ar² gegebenenfalls mit einem carbocyclischen, aromatischen oder nichtaromatischen Ring und einem 5- oder 6-gliedrigen, heterocyclischen aromatischen oder nicht-aromatischen Ring mit 1 oder 2 Heteroatomen, die ausgewählt sind unter O, S und N, kondensiert sein kann,
R¹ für H, C₃-C₆-Cycloalkyl oder C₁-C₆-Alkyl, das gegebenenfalls durch OH, OC₁-C₆-Alkyl oder Phenyl substituiert ist, steht;
die Reste R², die gleich oder verschieden sein können, für H oder C₁-C₆-Alkyl, das gegebenenfalls durch OH, OC₁-C₆-Alkyl oder Phenyl substituiert ist, stehen;
sowie deren Salze mit physiologisch verträglichen Säuren,
ausgenommen die Verbindungen 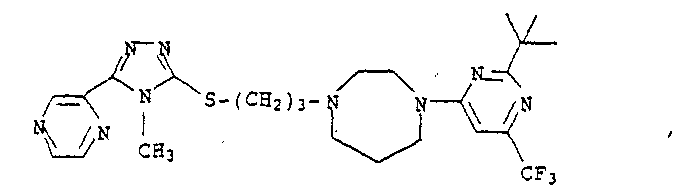 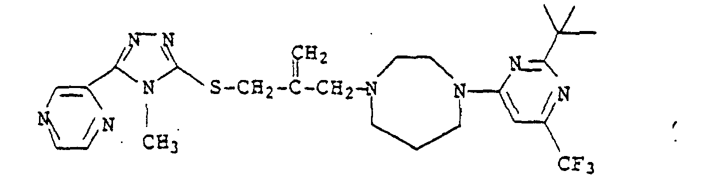 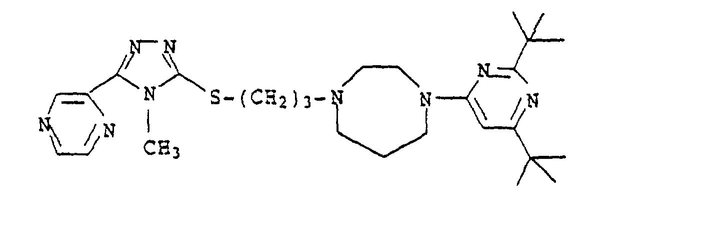 und deren Salze.

2. Verbindungen nach Anspruch 1 der Formel I, worin
Ar¹ für Phenyl, Naphthyl oder einen 5- oder 6-gliedrigen heterocyclischen aromatischen Ring mit 1 bis 3 Heteroatomen, die ausgewählt sind unter O, S und N, steht, wobei Ar¹ gegebenenfalls 1, 2, 3 oder 4 Substituenten aufweist, die unabhängig voneinander ausgewählt sind unter C₁-C₆-Alkyl, das gegebenenfalls durch OH, OC₁-C₆-Alkyl, Halogen oder Phenyl substituiert ist, C₁-C₆-Alkoxy, C₂-C₆-Alkenyl, C₂-C₆-Alkinyl, C₃-C₆-Cycloalkyl, Halogen, CN, COOR², NR²R², NO₂, SO₂R², SO₂NR²R² oder Phenyl, das gegebenenfalls durch C₁-C₆-Alkyl, OC₁-C₆-Alkyl, NR²R², CN, CF₃, CHF₂ oder Halogen substituiert ist, und wobei der erwähnte heterocyclische, aromatische Ring gegebenenfalls mit einem Phenylring kondensiert sein kann;
A für geradkettiges oder verzweigtes C₄-C₁₀ Alkylen oder geradkettiges oder verzweigtes C₃-C₁₀-Alkylen steht, das wenigstens eine Gruppe umfasst, die ausgewählt ist unter O, S, NR², CONR², COO, CO, einer Doppel- oder Dreifachbindung,
B für einen Rest der Formel steht: 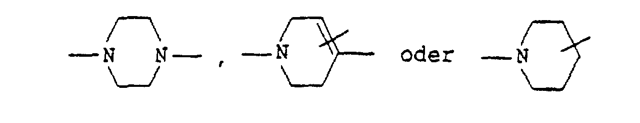
Ar² für Phenyl, Pyridyl, Pyrimidinyl oder Triazinyl steht, wobei Ar² gegebenenfalls ein bis vier Substituenten aufweisen kann, die unabhängig voneinander ausgewählt sind unter OR², C₂-C₆-Alkenyl, C₂-C₆-Alkinyl, C₁-C₆-Alkoxy-C₁-C₆-alkyl, Halogen-C₁-C₆-alkyl, Halogen-C₁-C₆-alkoxy, Halogen, CN, NO₂, SO₂R², NR²R², SO₂NR²R², einem 5- oder 6-gliedrigen carbocyclischen, aromatischen oder nicht-aromatischen Ring und einem 5- oder 6-gliedrigen, heterocyclischen aromatischen oder nicht-aromatischen Ring mit 1 oder 2 Heteroatomen, die ausgewählt sind unter O, S und N, wobei der carbocyclische oder heterocyclische Ring gegebenenfalls substituiert sein kann durch C₁-C₆-Alkyl, Phenyl, Phenoxy, Halogen, OC₁-C₆-Alkyl, OH, NO₂ oder CF₃ und wobei Ar² gegebenenfalls mit einem carbocyclischen oder heterocyclischen Ring der oben definierten Art kondensiert sein kann,
R¹ für H, C₃-C₆-Cycloalkyl oder C₁-C₆-Alkyl, das gegebenenfalls durch OH, OC₁-C₆-Alkyl oder Phenyl substituiert ist, steht;
die Reste R², die gleich oder verschieden sein können, für H oder C₁-C₆-Alkyl, das gegebenenfalls durch OH, OC₁-C₆-Alkyl oder Phenyl substituiert ist, stehen;
sowie deren Salze mit physiologisch verträglichen Säuren.

3. Verbindungen nach Anspruch 1 oder 2 der Formel I, worin A für C₄-C₁₀-Alkylen oder C₃-C₁₀-Alkylen steht, das gegebenenfalls wenigstens eine Gruppe Z umfasst, die ausgewählt ist unter O, S und einer Doppel- oder Dreifachbindung.

4. Verbindungen nach einem der vorhergehenden Ansprüche der Formel I, worin Ar¹ für Phenyl, Naphthyl, Pyrrolyl, Thienyl, Furanyl, Thiazolyl, Imidazolyl, Oxazolyl, Oxadiazolyl, Tetrazolyl, Isoxazolyl, Pyridinyl, Pyrazinyl, Pyrimidinyl, Benzthiophenyl, Indolyl oder Benzofuranyl steht, wobei Ar¹, wie in Anspruch 1 angegeben, substituiert oder kondensiert sein kann.

5. Verbindungen nach Anspruch 4 der Formel I, worin Ar¹ für Phenyl, Thienyl, Furanyl, Tetrazolyl, Pyrrolyl oder Pyrazinyl steht und, wie in Anspruch 1 angegeben, substituiert sein kann.

6. Verbindungen nach einem der vorhergehenden Ansprüche der Formel I, worin Ar¹ unsubstituiert ist oder 1, 2, 3 oder 4 Substituenten aufweist, die unabhängig voneinander ausgewählt sind unter CN, C₁-C₆-Alkyl, OH, OC₁-C₆-Alkyl, Phenyl und Halogen.

7. Verbindungen nach einem der vorhergehenden Ansprüche der Formel I, worin R¹ für H, C₁-C₆-Alkyl oder C₃-C₆-Cycloalkyl steht.

8. Verbindungen nach einem der vorhergehenden Ansprüche der Formel I, worin Ar² für Phenyl, Pyridinyl oder Pyrimidinyl steht, das gegebenenfalls einen oder zwei Substituenten aufweist, die unabhängig voneinander ausgewählt sind unter C₁-C₆-Alkyl, C₂-C₆-Alkinyl, Halogen, CN, Halogenalkyl, OAlkyl, NO₂, Phenyl, Pyrrolyl, Imidazolyl, Pyrazolyl, Thienyl, Indolyl, Cyclopentyl und Cyclohexyl.

9. Verbindungen nach Anspruch 8 der Formel I, worin der oder die Substituenten unabhängig voneinander ausgewählt sind unter C₁-C₆-Alkyl, Phenyl, NO₂, und Halogenalkyl, insbesondere CF₃, CHF₂ und CF₂Cl.

10. Verbindungen nach Anspruch 1 der Formel I, worin
Ar¹ für Phenyl steht, das gegebenenfalls durch C₁-C₆-Alkyl, OC₁-C₆-Alkyl, CN, Phenyl oder Halogen substituiert ist;
A die in Anspruch 2 angegebenen Bedeutungen besitzt;
B für 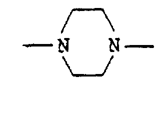 steht, und
Ar² für Pyrimidinyl steht, das gegebenenfalls substituiert ist durch C₁-C₆-Alkyl, Halogen-C₁-C₆-alkyl, Halogen-C₁-C₆-alkoxy, Pyrrolyl oder Indolyl.

11. Verbindungen nach Anspruch 10 der Formel I, worin
Ar¹ für Phenyl steht, das gegebenenfalls substituiert ist durch C₁-C₆-Alkyl, OC₁-C₆-Alkyl oder Halogen und
A für -S(CH₂)₃₋₁₀- oder -(CH₂)₄₋₁₀- steht.

12. Verbindungen nach einem der Ansprüche 1 bis 9 der Formel I, worin Ar¹ für einen 5- oder 6-gliedrigen heterocyclischen aromatischen Ring mit 1 bis 4 Heteroatomen, die unabhängig voneinander ausgewählt sind unter 0, S und N, steht, wobei der Ring wie in Anspruch 1 angegeben substituiert oder kondensiert sein kann, B für 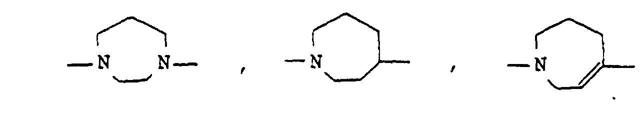 oder 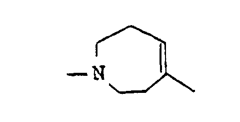 steht und A und Ar² die in Anspruch 1 angegebenen Bedeutungen besitzen.

13. Pharmazeutisches Mittel, enthaltend wenigstens eine Verbindung nach einem der Ansprüche 1 bis 12, gegebenenfalls zusammen mit physiologisch akzeptablen Trägern und/oder Hilfsstoffen.

14. Verwendung wenigstens einer Verbindung nach einem der Ansprüche 1 bis 12 zur Herstellung eines pharmazeutischen Mittels zur Behandlung von Erkrankungen, die auf Dopamin-D₃-Rezeptorantagonisten bzw. -agonisten ansprechen.

15. Verbindungen der Formel VIII 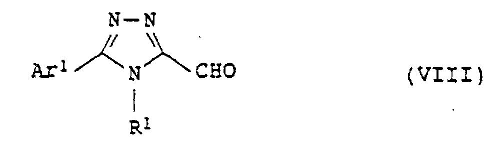 worin Ar¹ und R¹ für die in einem der Ansprüche 1, 2, 4 bis 7, 10 und 11 angegebenen Bedeutungen steht, ausgenommen Verbindungen, in denen R¹ für H steht und Ar¹ für Phenyl, p-Tolyl, 2-, 3-, 4-Pyridyl, p-Chlorphenyl oder p-Nitrophenyl steht.

## Claims

1. A triazole compound of the formula I 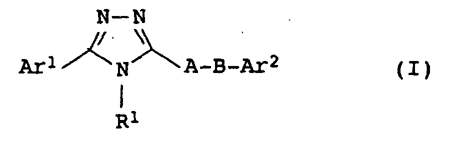 where
Ar¹ is phenyl, naphthyl or a 5- or 6-membered heterocyclic aromatic ring having from 1 to 4 heteroatoms which are selected, independently of one another, from O, S and N, where Ar¹ may have 1, 2, 3 or 4 substituents which are selected, independently of each other, from C₁-C₆-alkyl, which may be substituted by OH, OC₁-C₆-alkyl, halogen or phenyl, C₁-C₆-alkoxy, C₂-C₆-alkenyl, C₂-C₆-alkynyl, C₃-C₆-cycloalkyl, halogen, CN, COOR², NR²R², NO₂, SO₂R², SO₂NR²R² and phenyl, which may be substituted by C₁-C₆-alkyl, OC₁-C₆-alkyl, NR²R², CN, CF₃, CHF₂, or halogen, and where the heterocyclic, aromatic ring mentioned may also be fused to a phenyl ring;
A is straight-chain or branched C₄-C₁₀-alkylene or straight-chain or branched C₃-C₁₀-alkylene which comprises at least one Z group which is selected from O, S, NR², CONR², COO, CO, or a double or triple bond,
B is a radical of the formula: 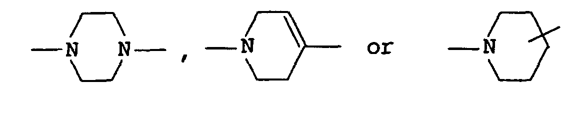 or if Ar¹ is the 5- or 6-membered heterocyclic aromatic ring which can be substituted as above, B is also a radical of the formulae 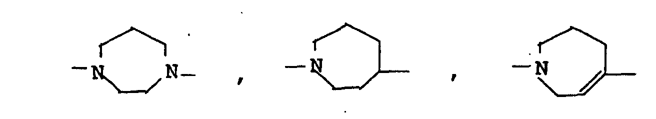 or 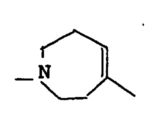
Ar² is phenyl, pyridyl, pyrimidinyl or triazinyl, where Ar² may have from 1 to 4 substituents which are selected, independently of each other, from OR², C₁-C₆-alkyl, C₂-C₆-alkenyl, C₂-C₆-alkynyl, C₁-C₆-alkoxy-C₁-C₆-alkyl, halogen-C₁-C₆-alkyl, halogen-C₁-C₆-alkoxy, halogen, CN, NO₂, SO₂R², NR²R², SO₂NR²R², a 5- or 6-membered carbocyclic, aromatic or non-aromatic ring and a 5- or 6-membered, heterocyclic aromatic or non-aromatic ring having 1 or 2 heteroatoms which are selected from O, S and N, where the carbocyclic or heterocyclic ring may be substituted by C₁-C₆-alkyl, phenyl, phenoxy, halogen, OC₁-C₆-alkyl, OH, NO₂ or CF₃ and/or fused to a phenyl ring and where Ar² may be fused to a carbocyclic, aromatic or non-aromatic ring and a 5- or 6-membered, heterocyclic aromatic or non-aromatic ring having 1 or 2 heteroatoms which are selected from O, S and N,
R¹ is H, C₃-C₆-cycloalkyl or C₁-C₆-alkyl which may be substituted by OH, OC₁-C₆-alkyl or phenyl;
the radicals R², which can be identical or different, are H or C₁-C₆-alkyl, which may be substituted by OH, OC₁-C₆-alkyl or phenyl;
and their salts with physiologically tolerated acids,
with the exception of the compounds 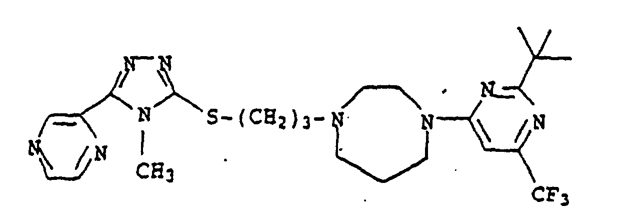 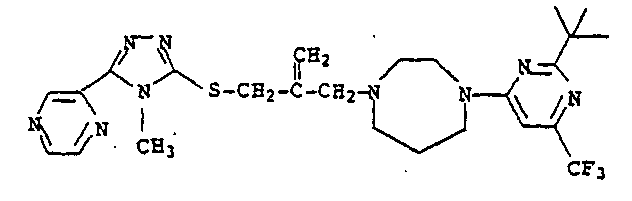 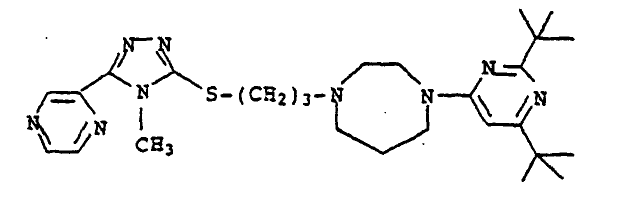 and their salts.

2. A compound of the formula I as claimed in claim 1, where
Ar¹ is phenyl, naphthyl or a 5- or 6-membered heterocyclic aromatic ring having from 1 to 3 heteroatoms which are selected from O, S and N, where Ar¹ may have 1, 2, 3 or 4 substituents which are selected, independently of each other, from C₁-C₆-alkyl, which may be substituted by OH, OC₁-C₆-alkyl, halogen or phenyl, C₁-C₆-alkoxy, C₂-C₆-alkenyl, C₂-C₆-alkynyl, C₃-C₆-cycloalkyl, halogen, CN, COOR², NR²R², NO₂, SO₂R², SO₂NR²R² or phenyl, which may be substituted by C₁-C₆-alkyl, OC₁-C₆-alkyl, NR²R², CN, CF₃, CHF₂, or halogen, and where the heterocyclic, aromatic ring mentioned may be fused to a phenyl ring;
A is straight-chain or branched C₄-C₁₀-alkylene or straight-chain or branched C₃-C₁₀-alkylene which comprises at least one group which is selected from O, S, NR², CONR², COO, CO, or a double or triple bond,
B is a radical of the formula: 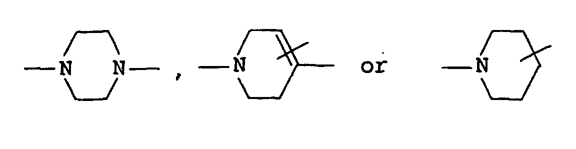
Ar² is phenyl, pyridyl, pyrimidinyl or triazinyl, where Ar² may have from 1 to 4 substituents which are selected, independently of each other, from OR², C₂-C₆-alkenyl, C₂-C₆-alkynyl, C₁-C₆-alkoxy-C₁-C₆-alkyl, halogen-C₁-C₆-alkyl, halogen-C₁-C₆-alkoxy, halogen, CN, NO₂, SO₂R², NR²R², SO₂NR²R², a 5- or 6-membered carbocyclic, aromatic or non-aromatic ring and a 5- or 6-membered, heterocyclic aromatic or non-aromatic ring having 1 or 2 heteroatoms which are selected from O, S and N, where the carbocyclic or heterocyclic ring may be substituted by C₁-C₆-alkyl, phenyl, phenoxy, halogen, OC₁-C₆-alkyl, OH, NO₂ or CF₃ and where Ar² may be fused to a carbocyclic or heterocyclic ring of the above-defined nature,
R¹ is H, C₃-C₆-cycloalkyl or C₁-C₆-alkyl which may be substituted by OH, OC₁-C₆-alkyl or phenyl;
the radicals R², which can be identical or different, are H or C₁-C₆-alkyl, which may be substituted by OH, OC₁-C₆-alkyl or phenyl;
and their salts with physiologically tolerated acids.

3. A compound of the formula I as claimed in claim 1 or 2, where A is C₄-C₁₀-alkylene or C₃-C₁₀-alkylene which may comprise at least one Z group which is selected from O, S and a double or triple bond.

4. A compound of the formula I as claimed in one of the preceding claims, where Ar¹ is phenyl, naphthyl, pyrrolyl, thienyl, furanyl, thiazolyl, imidazolyl, oxazolyl, oxadiazolyl, tetrazolyl, isoxazolyl, pyridinyl, pyrazinyl, pyrimidinyl, benzthiophenyl, indolyl or benzofuranyl, where Ar¹ can, as indicated in claim 1, be substituted or fused.

5. A compound of the formula I as claimed in claim 4, where Ar¹ is phenyl, thienyl, furanyl, tetrazolyl, pyrrolyl or pyrazinyl and can, as indicated in claim 1, be substituted.

6. A compound of the formula I as claimed in one of the preceding claims, where Ar¹ is unsubstituted or has 1, 2, 3 or 4 substituents which are selected, independently of each other, from CN, C₁-C₆-alkyl, OH, OC₁-C₆-alkyl, phenyl and halogen.

7. A compound of the formula I as claimed in one of the preceding claims, where R¹ is H, C₁-C₆-alkyl or C₃-C₆-cycloalkyl.

8. A compound of the formula I as claimed in one of the preceding claims, where Ar² is phenyl, pyridinyl or pyrimidinyl which may have one or two substituents which are selected, independently of each other, from C₁-C₆-alkyl, C₂-C₆-alkynyl, halogen, CN, haloalkyl, Oalkyl, NO₂, phenyl, pyrrolyl, imidazolyl, pyrazolyl, thienyl, indolyl, cyclopentyl and cyclohexyl.

9. A compound of a formula I as claimed in claim 8, wherein the substituent(s) is/are selected, independently of each other, from C₁-C₆-alkyl, phenyl, NO₂, and halogenoalkyl, in particular CF₃, CHF₂ and CF₂Cl.

10. A compound of the formula I as claimed in claim 1, where
Ar¹ is phenyl which may be substituted by C₁-C₆-alkyl, OC₁-C₆-alkyl, CN, phenyl or halogen;
A has the meanings given in claim 2;
B is 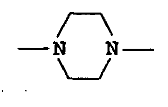 and
Ar² is pyrimidinyl which may be substituted by C₁-C₆-alkyl, halogen-C₁-C₆-alkyl, halogen-C₁-C₆-alkoxy, pyrrolyl or indolyl.

11. A compound of the formula I as claimed in claim 10, where
Ar¹ is phenyl which may be substituted by C₁-C₆-alkyl, OC₁-C₆-alkyl or halogen, and
A is -S(CH₂)₃₋₁₀- or -(CH₂)₄₋₁₀-.

12. A compound of the formula I as claimed in one of claims 1 to 9, where Ar¹ is a 5- or 6-membered heterocyclic aromatic ring having from 1 to 4 heteroatoms which are selected, independently of each other, from O, S and N, where the ring can, as indicated in claim 1, be substituted or fused, B is 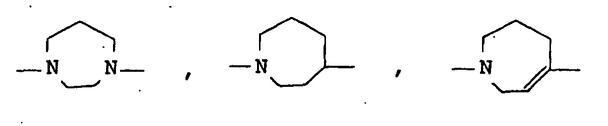 or 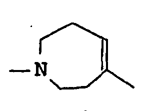 and A and Ar² have the meanings given in claim 1.

13. A pharmaceutical which comprises at least one compound as claimed in one of claims 1 to 12, with or without physiologically acceptable carrier substances and/or auxiliary substances.

14. The use of at least one compound as claimed in one of claims 1 to 12 for preparing a pharmaceutical for the treatment of diseases which respond to dopamine D₃ receptor antagonists or dopamine D₃ agonists.

15. A compound of the formula VIII 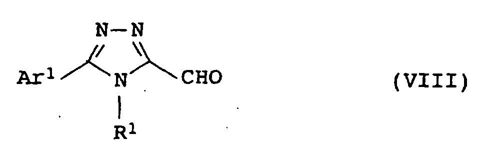 where Ar¹ and R¹ have the meanings given in one of the claims 1, 2, 4 to 7, 10 and 11, with the exception of compounds in which R¹ is H and Ar¹ is phenyl, p-tolyl, 2-, 3-, 4-pyridyl, p-chlorophenyl or p-nitrophenyl.

## Revendications

1. Triazoles répondant à la formule I 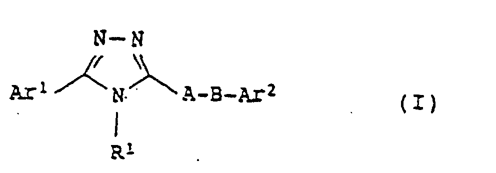 dans laquelle
Ar¹ représente un groupe phényle, naphtyle ou un hétérocycle aromatique à cinq ou six chaînons contenant un à quatre hétéroatomes choisis, indépendamment les uns des autres parmi O, S et N, Ar¹ portant le cas échéant un, deux, trois ou quatre substituants choisis, indépendamment les uns des autres, parmi les groupes alkyle en C1-C6, eux-mêmes éventuellement substitués par OH, Oalkyle en C1-C6, des halogènes ou des groupes phényle, les groupes alcoxy en C1-C6, alcényle en C2-C6, alcynyle en C2-C6, cycloalkyle en C3-C6, les halogènes, les groupes CN, COOR², NR²R², NO², SO₂R², SO₂NR²R² et phényle, lui-même éventuellement substitué par des groupes alkyle en C1-C6, Oalkyle en C1-C6, NR²R², CN, CF₃, CHF₂ ou des halogènes, l'hétérocycle aromatique en question pouvant le cas échéant être condensé avec un cycle phényle ;
A représente un groupe alkylène à chaîne droite ou ramifiée en C4-C10 ou un groupe alkylène à chaîne droite ou ramifiée en C3-C10 comprenant au moins une partie Z choisie parmi O, S NR², CONR², COO, CO, une double liaison ou une triple liaison,
B représente un groupe de formule 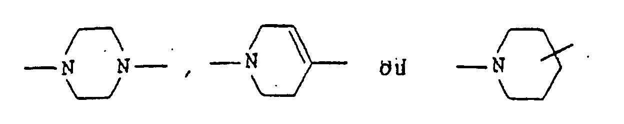 ou bien, lorsque Ar¹ représente l'hétérocycle aromatique à cinq ou six chaînons qui peut porter les substituants indiqués ci-dessus, B peut également représenter un groupe répondant à l'une des formules 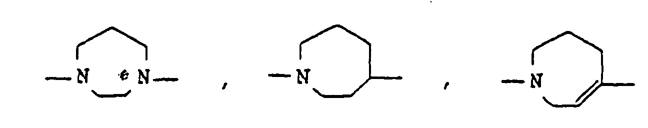 ou 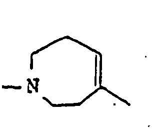
Ar² représente un groupe phényle, pyridyle, pyrimidinyle ou triazinyle, Ar² pouvant éventuellement porter un à quatre substituants choisis, indépendamment les uns des autres, parmi OR², les groupes alkyle en C1-C6, alcényle en C2-C6, alcynyle en C2-C6, (alcoxy en C1-C6)alkyle en C1-C6, halogénoalkyle en C1-C6, halogénoalcoxy en C1-C6, les halogènes, les groupes CN, NO₂, SO₂R², NR²R², SO₂NR²R², un cycle homogène aromatique ou non aromatique à cinq ou six chaînons et un hétérocycle aromatique ou non aromatique à cinq ou six chaînons contenant un ou deux hétéroatomes choisis parmi O, S et N, le cycle homogène ou hétérogène pouvant éventuellement porter des substituants alkyle en C1-C6, phényle, phénoxy, des halogènes, des groupes Oalkyle en C1-C6, OH, NO₂ ou CF₃ et/ou pouvant être condensés avec un cycle phényle, Ar² pouvant éventuellement être condensé avec un cycle homogène aromatique ou non aromatique et un hétérocycle aromatique ou non aromatique à cinq ou six chaînons contenant un ou deux hétéroatomes choisis parmi O, S et N,
R¹ représente H, un groupe cycloalkyle en C3-C6 ou alkyle en C1C6 qui peut le cas échéant porter des substituants OH, Oalkyle en C1-C6 où phényle ;
les symboles R², ayant des significations identiques ou différentes, représentent chacun H ou un groupe alkyle en C1-C6 qui peut porter éventuellement des substituants OH, Oalkyle en C1-C6 ou phényle ; et leurs sels d'acides acceptables pour l'usage pharmaceutique,
à l'exception des composés 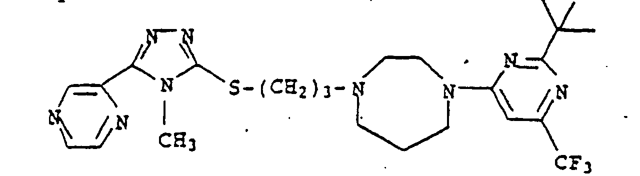 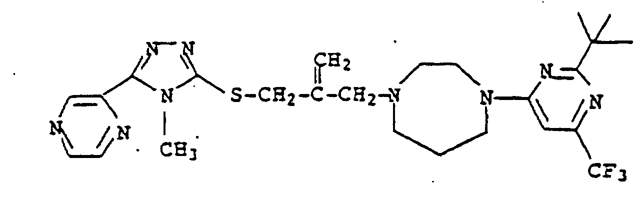 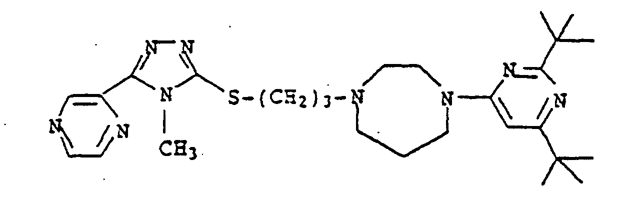 et de leurs sels.

2. Composés selon la revendication 1, répondant à la formule I dans laquelle
Ar¹ représente un groupe phényle, naphtyle ou un hétérocycle aromatique à cinq ou six chaînons contenant un à trois hétéroatomes choisis parmi O, S et N, Ar¹ pouvant éventuellement porter un, deux, trois ou quatre substituants choisis, indépendamment les uns des autres, parmi les groupes alkyle en C1-C6, eux-mêmes éventuellement substitués par OH, Oalkyle en C1-C6, des halogènes ou des groupes phényle, un groupe alcoxy en C1-C6, alcényle en C2-C6, alcynyle en C2-C6, cycloalkyle en C3-C6, un halogène, un groupe CN, COOR², NR²R², NO₂, SO₂R², SO₂R²R² ou phényle, ce dernier pouvant éventuellement porter des substituants alkyle en C1-C6, Oalkyle en C1-C6, NR²R², CN, CF₃, CHF₂ ou halogéno, l'hétérocycle aromatique en question pouvant le cas échéant être condensé avec un cycle phényle ;
A représente un groupe alkylène à chaîne droite ou ramifiée en C4-C10 ou un groupe alkylène à chaîne droite ou ramifiée en C3-C10 comprenant au moins une partie choisie parmi O, S, NR², CONR², COO, CO, une double liaison ou une triple liaison,
B représente un groupe de formule : 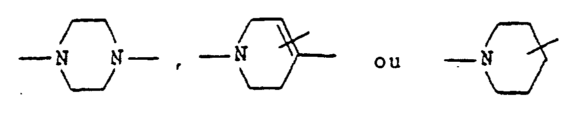
Ar² représente un groupe phényle, pyridyle, pyrimidinyle ou triazinyle, Ar² pouvant éventuellement porter un à quatre substituants choisis, indépendamment les uns des autres, parmi OR², les groupes alcényle en C2-C6, alcynyle en C2-C6, (alcoxy en C1-C6)alkyle en C1-C6, halogénoalkyle en C1-C6, halogénoalcoxy en C1-C6, les halogènes, les groupes CN, NO₂, SO₂R², NR²R², SO₂NR²R², un cycle homogène aromatique ou non aromatique à cinq ou six chaînons et un hérérocycle aromatique ou non aromatique à cinq ou six chaînons contenant un ou deux hétéroatomes choisis parmi O, S et N, le cycle homogène ou hétérogène pouvant le cas échéant porter des substituants alkyle en C1-C6, phényle, phénoxy, halogéno, Oalkyle en C1-C6, OH, NO₂ ou CF₃, Ar² pouvant éventuellement être condensé avec un cycle homogène ou hétérogène du type défini ci-dessus,
R¹ représente H, un groupe cycloalkyle en C3-C6 ou alkyle en C1-C6 portant éventuellement des substituants OH, Oalkyle en C1-C6 ou phényle ;
les symboles R², ayant des significations identiques ou différentes, représentent chacun H ou un groupe alkyle en C1-C6 portant éventuellement des substituants OH, Oalkyle en C1-C6 ou phényle ;
et leurs sels d'acides acceptables pour l'usage pharmaceutique.

3. Composés selon l'une des revendications 1 ou 2, répondant à la formule I dans laquelle A représente un groupe alkylène en C4-C10 ou alkylène en C3-C10, ce dernier contenant le cas échéant une partie Z choisie parmi O, S, une double liaison ou une triple liaison.

4. Composés selon l'une des revendications qui précèdent, répondant à la formule I, dans laquelle Ar¹ représente un groupe phényle, naphtyle, pyrrolyle, thiényle, furannyle, thiazolyle, imidazolyle, oxazolyle, oxadiazolyle, tétrazolyle, isoxazolyle, pyridinyle, pyrazinyle, pyrimidinyle, benzothiophényle, indolyle ou benzofurannyle, Ar¹ pouvant être substitué ou condensé comme indiqué dans la revendication 1.

5. Composés selon la revendication 4, répondant à la formule I, dans laquelle Ar¹ représente un groupe phényle, thiényle, furannyle, tétrazolyle, pyrrolyle ou pyrazinyle, qui peut être substitué comme indiqué dans la revendication 1.

6. Composés selon l'une des revendications qui précèdent, répondant à la formule I, dans laquelle Ar¹ est non substitué ou porte un, deux, trois ou quatre substituants choisis, indépendamment les uns des autres, parmi les groupes CN, alkyle en C1-C6, OH, Oalkyle en C1-C6, phényle et les halogènes.

7. Composés selon l'une des revendications qui précèdent, répondant à la formule I dans laquelle R¹ représente H, un groupe alkyle en C1-C6 ou cycloalkyle en C3-C6.

8. Composés selon l'une des revendications qui précèdent, répondant à la formule I, dans laquelle Ar² représente un groupe phényle, pyridinyle ou pyrimidinyle portant éventuellement un ou deux substituants choisis, indépendamment les uns des autres, parmi les groupes alkyle en C1-C6, alcynyle en C2-C6, les halogènes, les groupes CN, halogénoalkyle, Oalkyle, NO₂, phényle, pyrrolyle, imidazolyle, pyrazolyle, thiényle, indolyle, cyclopentyle et cyclohexyle.

9. Composés selon la revendication 8, répondant à la formule I dans laquelle le ou les substituants sont choisis, indépendamment les uns des autres, parmi les groupes alkyle en C1-C6, phényle, NO₂ et halogénoalkyle, plus spécialement CF₃, CHF₃ et CF₂Cl.

10. Composés selon la revendication 1, répondant à la formule I, dans laquelle
Ar¹ représente un groupe phényle portant éventuellement des substituants alkyle en C1-C6, Oalkyle en C1-C6, CN, phényle ou halogéno ;
A a les significations indiquées dans la revendication 2 ;
B représente 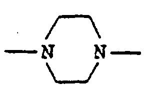 et,
Ar² représente un groupe pyrimidinyle portant éventuellement des substituants alkyle en C1-C6, halogénoalkyle en C1-C6, halogénoalcoxy en C1-C6, pyrrolyle ou indolyle.

11. Composés selon la revendication 10, répondant à la formule I, dans laquelle
Ar¹ représente un groupe phényle portant éventuellement des substituants alkyle en C1-C6, Oalkyle en C1-C6 ou halogéno et
A représente -S(CH₂)₃₋₁₀ ou -(CH₂)₄₋₁₀.

12. Composés selon l'une des revendications 1 à 9, répondant à la formule I dans laquelle Ar¹ représente un hétérocycle aromatique à cinq ou six chaînons contenant un à quatre hétéroatomes choisis, indépendamment les uns des autres, parmi O, S et N, le cycle pouvant être substitué ou condensé comme indiqué dans la revendication 1, B représente 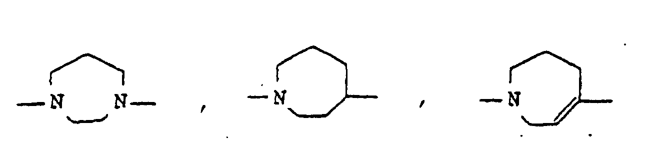 ou 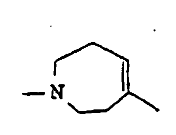 et A et Ar² ont les significations indiquées dans la revendication 1.

13. Produit pharmaceutique contenant au moins un composé selon l'une des revendications 1 à 12, les cas échéant avec des véhicules et/ou produits auxiliaires acceptables pour l'usage pharmaceutique.

14. Utilisation d'au moins un composé selon l'une des revendications 1 à 12 pour la préparation d'un produit pharmaceutique prévu pour le traitement de maladies demandant des antagonistes ou agonistes des récepteurs D₃ de la dopamine.

15. Composés de formule VIII 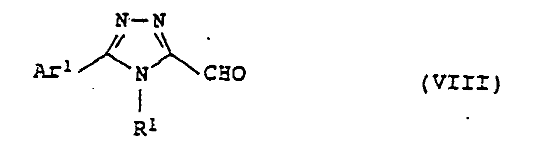 dans laquelle Ar¹ et R¹ ont les significations indiquées dans les revendications 1, 2, 4 à 7, 10 et 11, à l'exception des composés pour lesquels R¹ représente H et Ar¹ un groupe phényle, p-tolyle, 2-, 3-, 4-pyridyle, p-chlorophényle ou p-nitrophényle.
